(19) Europäisches Patentamt
European Patent Office
Office européen des brevets

(11) Publication number: 0 170 412 B1

(12) **EUROPEAN PATENT SPECIFICATION**

(45) Date of publication of patent specification: 28.06.89

(21) Application number: 85304631.6

(22) Date of filing: 28.06.85

(51) Int. Cl.⁴: C 07 D 517/06, A 61 K 31/415, C 07 D 345/00 // (C07D517/06, 345:00, 231:00)

(54) **Benzoselenino[4,3,2-cd]indazole compounds, compositions comprising the compounds and processes for producing the compounds.**

(30) Priority: 29.06.84 US 626170

(43) Date of publication of application: 05.02.86 Bulletin 86/06

(45) Publication of the grant of the patent: 28.06.89 Bulletin 89/26

(84) Designated Contracting States: AT BE CH DE FR GB IT LI LU NL SE

(56) References cited:
EP-A-0 103 381
EP-A-0 114 002
DE-A-1 816 086

(73) Proprietor: WARNER-LAMBERT COMPANY
201 Tabor Road
Morris Plains New Jersey 07950 (US)

(72) Inventor: Berman, Ellen M.
3505 Green Brier Apt. 30C
Ann Arbor Michigan 48105 (US)
Inventor: Showalter, Hollis H. D.
900 Patricia
Ann Arbor Michigan 48103 (US)
Inventor: Gregor, Vlad E.
2231-2 Cram Place
Ann Arbor Michigan 48105 (US)

(74) Representative: Jones, Michael Raymond et al
HASELTINE LAKE & CO. Hazlitt House
28 Southampton Buildings Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 170 412 B1

**Description**

The invention relates to novel substituted benzoselenino[4,3,2-*cd*]indazoles, to methods for their production and to pharmaceutical compositions comprising the compounds. The compounds of the invention have pharmacological properties and are useful antibacterial agents, antifungal agents, and antitumor agents.

US—3505341 relates to benzoselenino[4,3,2-*cd*]indazole compounds having antibacterial, schistosomicidal and antitrichomonal activities.

The benzoselenino[4,3,2-*cd*]indazole ring system is new and is illustrated by the formula

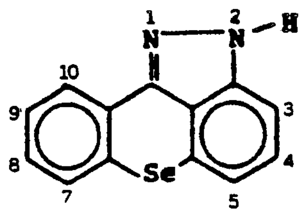

The invention in one aspect relates to benzoselenino[4,3,2-*cd*]indazole compounds having, in free base form, the structural Formula 1:

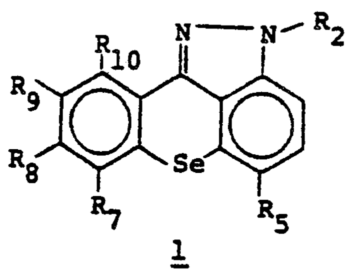

1

and to the pharmaceutically acceptable salts, thereof, wherein the substituents $R_7$, $R_8$, $R_9$, and $R_{10}$ represent hydrogen, hydroxy or alkoxy of from one to four carbon atoms, hereinafter referred to as lower alkoxy; wherein $R_2$ is ANR′R″ wherein A is a straight or branched alkylene chain of from two to five carbon atoms, optionally substituted with hydroxyl; wherein R′ and R″ are hydrogen or straight or branched alkyl of from one to four carbon atoms, optionally substituted with hydroxyl, R′ and R″ taken together also representing

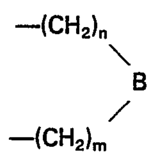

wherein n and m are each an integer from two to three and B is a direct bond or O, S, or NR‴ wherein R‴ is hydrogen or straight or branched alkyl of from one to four carbon atoms, optionally substituted with hydroxyl; and wherein $R_5$ is nitro, $NH_2$, $NHR_2$, or NHR⁗ wherein R⁗ is from one to four carbon atoms acyl, chloroacetyl,

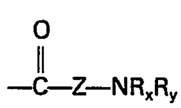

wherein $R_x$ and $R_y$ are each hydrogen or straight or branched one to four carbon atoms alkyl optionally substituted with hydroxyl and Z is a straight or branched alkylene chain of from one to four carbon atoms or R⁗ is the group

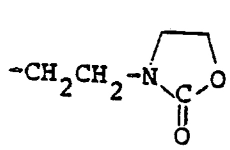

The compounds of the invention form pharmaceutically acceptable salts with both organic and

inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic, isethionic, lactic, gluconic, glucuronic, sulfamic, benzoic, tartaric or pamoic acid. The salts are prepared by contacting the free base form with an equivalent amount of the desired acid in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention.

The compounds of the invention can exist in unsolvated as well as solvated forms, including hydrated forms. In general, the solvated forms with pharmaceutically acceptable solvents such as water or ethanol, are equivalent to the unsolvated forms for purposes of the invention.

The term halogen as used herein is intended to include fluorine, chlorine, bromine, and iodine.

The invention in another aspect relates to benzoselenino[4,3,2-*cd*]indazole compounds having the structural Formula 2:

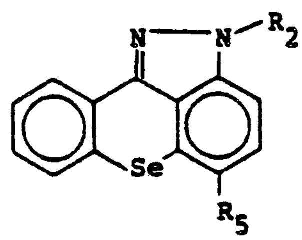

2

and the pharmaceutically acceptable salts thereof; wherein $R_2$ and $R_5$ have the above meanings.

The invention in another aspect relates to benzoselenino[4,3,2-*cd*]indazole compounds having the structural Formula 3:

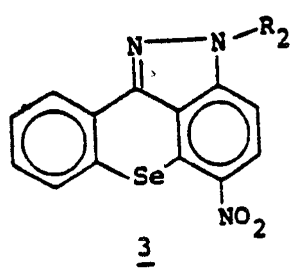

3

and the pharmaceutically acceptable salts thereof; wherein $R_2$ has the above meaning.

The invention is another aspect relates to benzoselenino[4,3,2-*cd*]indazole compounds having the structural Formula 4:

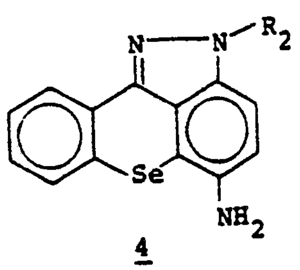

4

and the pharmaceutically acceptable salts thereof; wherein $R_2$ has the above meaning.

The invention in another embodiment relates to benzoselenio[4,3,2-*cd*]indazole compounds having the structural Formula 5:

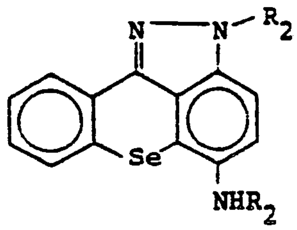

5

and the pharmaceutically acceptable salts thereof; wherein $R_2$ has the above meaning.

The invention in another embodiment relates to benzoselenino[4,3,2-*cd*]indazole compounds having the structural Formula 6:

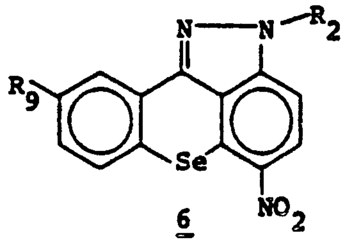

6

and the pharmaceutically acceptable salts thereof; wherein $R_2$ has the above meaning and $R_9$ is lower alkoxy.

The invention in another embodiment relates to benzoselenino[4,3,2-*cd*]indazole compounds having the structural Formula 7:

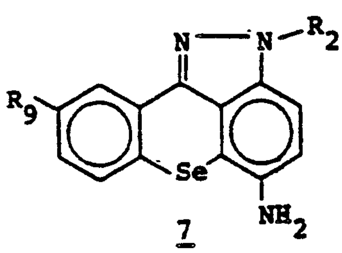

7

and the pharmaceutically acceptable salts thereof; where $R_2$ has the above meaning and $R_9$ is lower alkoxy.

The invention in another embodiment relates to benzoselenino[4,3,2-*cd*]indazole compounds having the structural Formula 8:

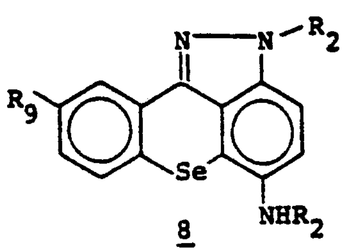

8

and the pharmaceutically acceptable salts thereof; where $R_2$ has the above meaning and $R_9$ is lower alkoxy.

The invention in another embodiment relates to benzoselenino[4,3,2-*cd*]indazole compounds having the structural Formula 9:

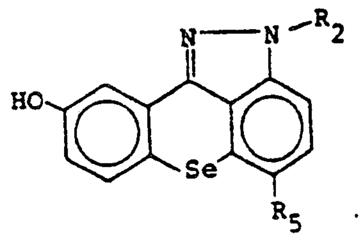

9

and the pharmaceutically acceptable salts thereof; wherein $R_2$ and $R_5$ have the above meaning.

The invention in another embodiment relates to benzoselenino[4,3,2-*cd*]indazole compounds having the structural Formula 10:

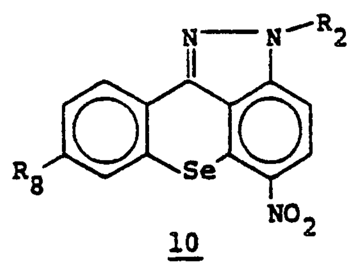

10

and the pharmaceutically acceptable salts thereof; wherein $R_2$ has the above meaning and $R_8$ is lower alkoxy.

The invention in another embodiment relates to benzoselenino[4,3,2-*cd*]indazole compounds having the structural Formula 11:

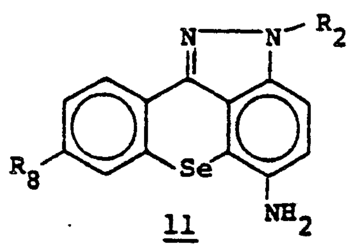

11

and the pharmaceutically acceptable salts thereof; wherein $R_2$ has the above meaning and $R_8$ is lower alkoxy.

The invention in another embodiment relates to benzoselenino[4,3,2-*cd*]indazole compounds having the structural Formula 12:

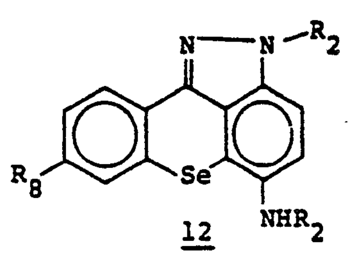

12

and the pharmaceutically acceptable salts thereof; wherein $R_2$ has the above meaning and $R_8$ is lower alkoxy.

The invention in another embodiment relates to benzoselenino[4,3,2-*cd*]indazole compounds having the structural Formula 13:

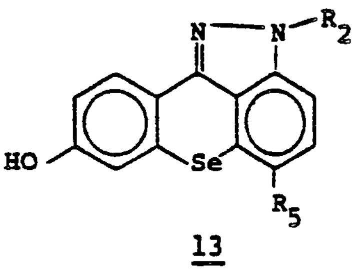

13

and the pharmaceutically acceptable salts thereof; wherein $R_2$ and $R_5$ have the above meaning.

The invention in another aspect relates to compounds that are most preferred for their pharmacological properties, these compounds have the following names:

*N,N*-diethyl-5-nitro-2*H*-[1]benzoselenino[4,3,2-*cd*]indazole-2-ethanamine;
5-amino-*N,N*-diethyl-2*H*-[1]benzoselenino[4,3,2-*cd*]indazole-2-ethanamine;
*N*-[2-[2-(diethylamino)ethyl]-2*H*-[1]-benzoselenino[4,3,2-*cd*]indazol-1,2-ethanediamine;
*N*-[2-[2-(diethylamino)ethyl]-9-methoxy-2*H*-[1]benzoselenino[4,3,2-*cd*]indazol-5-yl]ethanediamine;
2-[[2-[[2-[2-(diethylamino)ethyl]-2*H*-[1]benzoselenino[4,3,2-*cd*]indazol-5-yl]amino]ethyl]amino]-ethanol;
2-[[2-[5-[[2-[(2-hydroxyethyl)amino]ethyl]amino]-2*H*-[1]benzoselenino[4,3,2-*cd*]indazol-2-yl]ethyl]-amino]ethanol;
*N,N*-diethyl-5-[[2-(diethylamino)ethyl]amino]-2*H*-[1]benzoselenino[4,3,2-*cd*]indazole-2-ethanamine;
*N*-[2-[2-(diethylamino)ethyl]-2*H*-[1]benzoselenino[4,3,2-*cd*]indazol-5-yl]-1,3-propanediamine;
5-[(2-aminoethyl)amino]-2-[2-(1-pyrrolidinyl)ethyl]-2*H*-[1]benzoselenino[4,3,2-*cd*]indazole;
2-[2-(diethylamino)ethyl]-5-nitro-2*H*-[1]benzoselenino[4,3,2-*cd*]indazol-9-ol;
2-[2-(diethylamino)ethyl]-5-nitro-2*H*-[1]benzoselenino[4,3,2-*cd*]indazol-8-ol;
5-[(2-aminoethyl)amino]-2-[2-(diethylamino)ethyl]-2*H*-[1]benzoselenino[4,3,2-*cd*]indazol-9-ol;
5-[(2-aminoethyl)amino]-2-[2-(diethylamino)ethyl]-2*H*-[1]benzoselenino[4,3,2-*cd*]indazol-8-ol;
2-[2-(diethylamino)ethyl]-5-[[2-[(2-hydroxyethyl)amino]ethyl]amino]-2*H*-[1]benzoselenino[4,3,2-*cd*]-indazol-8-ol;
2-[2-(diethylamino)ethyl]-5-[[2-[(2-hydroxyethyl)amino]ethyl]amino]-2*H*-[1]benzoselenino[4,3,2-*cd*]-indazol-9-ol; and the pharmaceutically acceptable salts thereof.

Certain of the compounds of the invention are also useful as intermediates in the preparation of the preferred species; more specifically, those compounds of structure *1* wherein $R_5$ represents $NO_2$ or $NH_2$ and those compounds such as those of structures *7, 8,* and *9* described below which contain protecting groups.

The invention also includes further novel intermediates of the Formula 14:

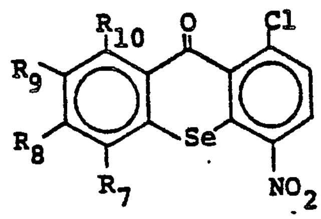

14

wherein $R_7$, $R_8$, $R_9$ and $R_{10}$ are hydrogen, hydroxy or alkoxy of from 1 to 4 carbon atoms. Preferred intermediates also include compounds of the formula 15:

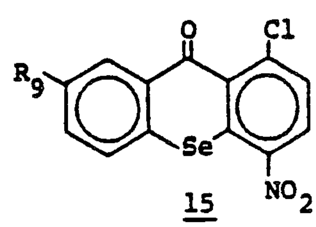

15

wherein $R_9$ is hydrogen, hydroxy or alkoxy of one to four carbon atoms.

PROCESS FOR PREPARING THE COMPOUNDS

The invention in one process aspect comprises a process for preparing compounds having the structural Formula 1a:

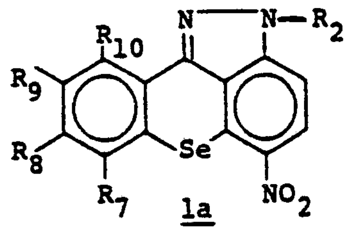

1a

by reacting a 1-halo-4-nitro-9*H*-selenoxanthen-9-one and $R_2$-substituted hydrazine having the structural formula $H_2NNHR_2$, wherein $R_2$, $R_7$, $R_8$, $R_9$, and $R_{10}$ have the above meaning. The reaction conditions can be varied widely. The reaction is usually carried out in a solvent at temperatures between about 25 to about 140°C. A suitable solvent is xylene, pyridine or DMF.

The requisite hydrazines are prepared by reaction of hydrazine with the appropriate alkyl halide, $XR_2$, wherein $R_2$ has the above meaning [*J. Med. Chem., 7,* 403 (1964)], or other methods known in the art as described below.

The 1-halo-4-nitro-9*H*-selenoxanthen-9-ones are prepared by cyclization of the corresponding 2-[[5-chloro-2-nitrophenyl)seleno]benzoic acid preferably with phosphorous pentoxide and methanesulfonic acid or alternatively with phosphorus pentoxide and hexamethyldisiloxane. The requisite benzoic acids are prepared by the reaction of an *o*-selenocyanobenzoic acid with a 2,4-dichloronitrobenzene. The process is illustrated as follows:

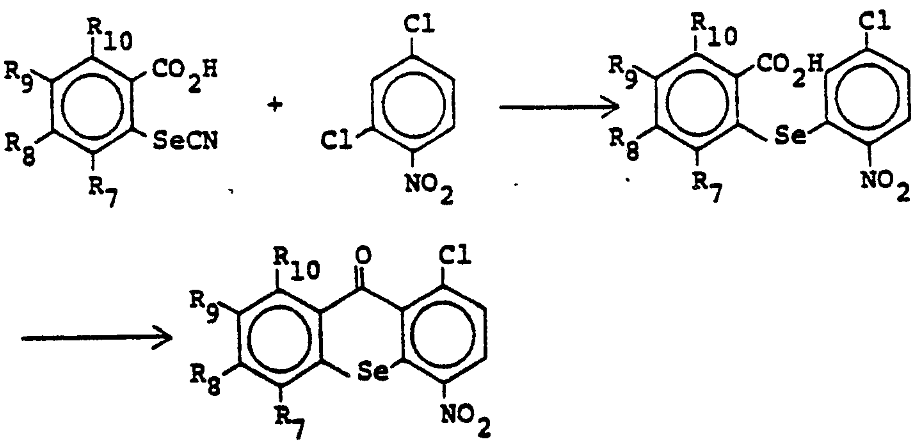

The invention in another aspect comprises a process for preparing compounds having the structural Formula 1b:

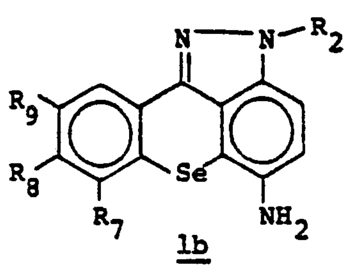

1b

by subjecting a compound having the structural Formula 1a:

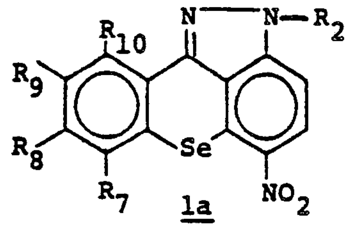

1a

to reduction, wherein $R_2$, $R_7$, $R_8$, $R_9$, and $R_{10}$ have the above meaning. The reduction is carried out by suitable means, preferably by hydrogenation at room temperature using a palladium/charcoal catalyst in a solvent such as methanol or acetic acid, acetic acid being preferred.

The invention in another aspect comprises a process for preparing compounds having the structural Formula 1c:

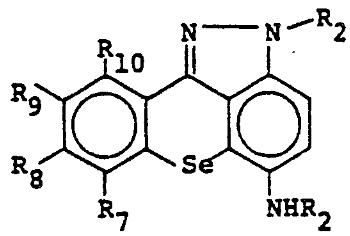

1c

by reacting a compound having the structural Formula 1b:

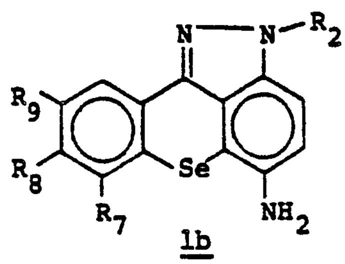

1b

with a haloalkylamine having the formula $XR_2$, wherein $R_2$, $R_7$, $R_8$, $R_9$ and $R_{10}$ have the above meaning and X is a halogen. Where $R_2$ includes a sensitive group, this is appropriately protected prior to the reaction by a protective group. The reaction is carried out in the absence of solvent or in a suitable unreactive solvent such as $CHCl_3$ or DMF. In the absence of solvent, the reaction temperature may be about 150°C. With solvent, reflux temperature is used, for example, from about 60 to about 150°C. Bases such as $Et_3N$ or $K_2CO_3$ may be employed as acid scavengers but are not essential.

In another aspect, the invention comprises a process for preparing compounds having the structural Formula 1c above, by reacting a compound having the Formula 1b above with the appropriately substututed aldehyde or acetal, ketone or ketal at reflux temperature, for example, from about 65 to about 140°C, and reducing the resulting Schiff base with a suitable reducing agent such as $NaBH_4$ or $NaBH_3CN$; wherein $R_2$, $R_7$, $R_8$, $R_9$, and $R_{10}$ have the above meaning.

In one preferred embodiment, the invention comprises a process for preparing benzoselenino[4,3,2-*cd*]indazole compounds having the structural Formula 1:

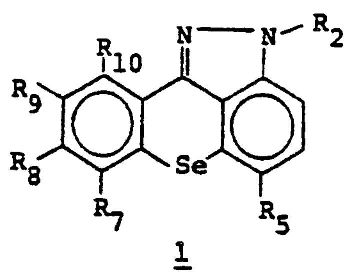

1

which comprises reacting an optionally substituted 1-chloro-4-nitro-9*H*-selenoxanthen-9-one and an $R_2$-

substituted hydrazine to form the compounds having the structural formula *1* where $R_5$ is nitro and, if desired, converting said compounds by reduction to compounds having the structural formula *1* where $R_5$ is $NH_2$ and if further desired converting said compounds to compounds having the structural formula *1* where $R_5$ is $NHR_2$ by alkylation with a haloalkylamine having the formula $XR_2$, optionally after covering sensitive groups with protective groups; or by monoacylating with a reactive derivative of an acylating agent of formula R''''COOH and reducing the acylated product, optionally after covering sensitive groups with protecting groups; and if desired, removing the protecting groups by hydrolysis or reduction; and isolating the product in free base or acid addition salt form; wherein X is chloro or bromo and $R_2$, $R_3$, R'''', $R_5$, $R_7$, $R_8$, $R_9$, and $R_{10}$ have the above meaning.

In another embodiment, the invention comprises a process for preparing benzoselenino[4,3,2-*cd*]-indazole compounds having in free base form the structural formula:

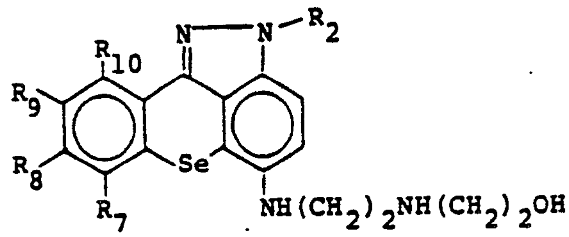

by reacting a compound having the structural formula:

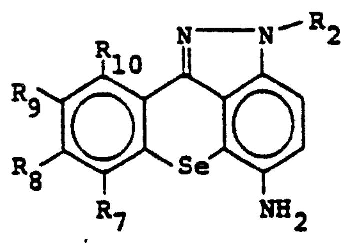

wherein $R_3$, $R_7$, $R_8$, $R_9$, and $R_{10}$ are hydrogen, hydroxyl, lower alkoxy, or benzyloxy, or *p*-halo or *p*-methoxy substituted benzyloxy, with 3-(β-haloethyl)-2-oxazolidinone to obtain an oxazolidinone compound having the structural formula:

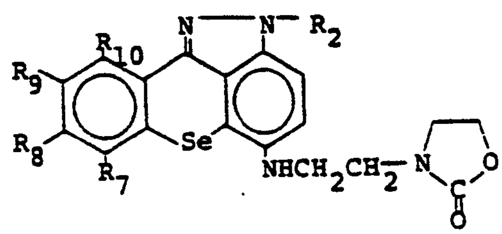

as defined above and subjecting the latter compound to alkaline hydrolysis to obtain a compound having the structural formula:

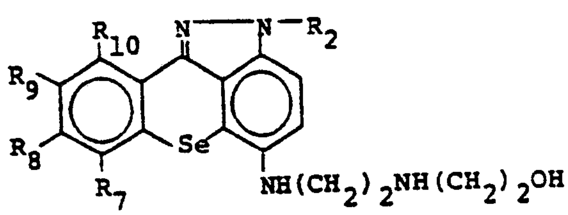

if necessary, removing any benzyloxy groups by hydrogenolysis, and isolating the product in free base or acid addition salt form. The invention also contemplates the embodiment in which one or more of $R_3$, $R_7$, $R_8$, $R_9$, and $R_{10}$ are benzyloxy or alkoxy wherein the benzyloxy and alkoxy groups are removed by hydrogenolysis or treatment with boron tribromide.

In another embodiment, the invention comprises a process for preparing compounds having the structural Formula 1c:

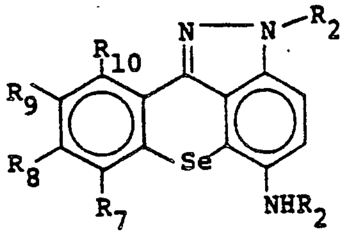

1c

by subjecting a compound having the structural Formula 16:

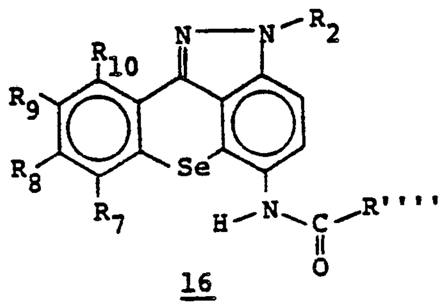

16

to reduction, wherein $R_2$, $R_7$, $R_8$, $R_9$, $R_{10}$ and R'''' have the above meaning. One uses a suitable reducing agent, preferably in an ethereal solvent such as ether, dioxane, THF or ethylene glycol dimethyl ether. Typically $LiAlH_4$ is employed, but other reducing agents, for example, aluminum derivatives such as $AlH_3$ may be used.

In still another embodiment, the invention comprises a process for preparing compounds having the structural Formula 6:

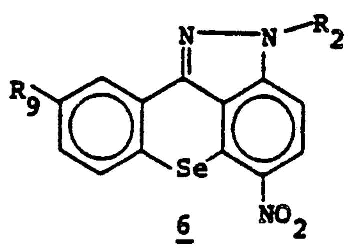

6

by reacting a compound having the structural Formula 15:

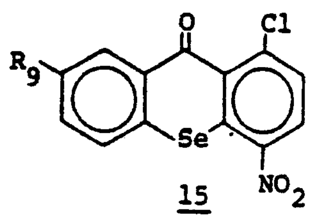

15

with a substituted hydrazine having the structural formula $H_2NNHR_2$; wherein $R_2$ has the above meaning and $R_9$ is hydroxy or alkoxy of from one to four carbon atoms and may also be benzyloxy of halo- or methoxy-substituted benzyloxy.

In still another embodiment, the invention comprises a process for preparing compounds having the structural Formula 7:

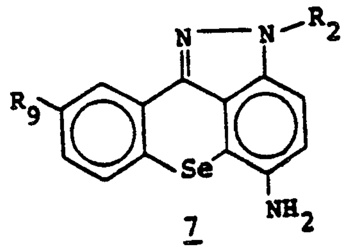

7

by subjecting to reduction a compound having the structural Formula 6:

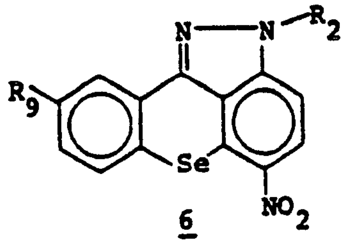

6

wherein $R_2$ has the above meaning and $R_9$ is hydroxy or alkoxy of from one to four carbon atoms and may also be benzyloxy or halo- or methoxy-substituted benzyloxy. Preferably, the reduction is carried out in AcOH using Pd/C as the catalyst for the methoxy substituted compounds and in MeOH using Raney nickel as the catalyst for the benzyloxy substituted compounds.

The invention also comprises a process for the preparing compounds having the structural Formula 8:

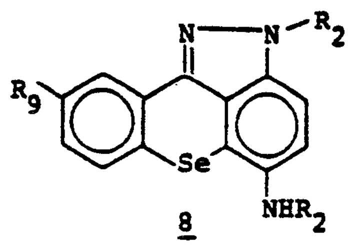

8

by alkylating a compound having the structural Formula 7:

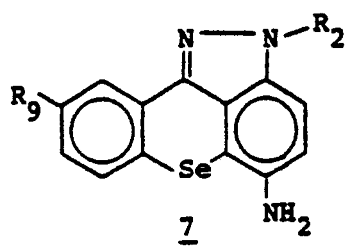

7

wherein $R_2$ has the above meaning and $R_9$ is hydroxy or alkoxy of from one to four carbon atoms and may also be benzyloxy or *p*-halo- or *p*-methoxy-substituted benzyloxy.

In another embodiment, the invention comprises a process for preparing compounds having the structural Formula 9:

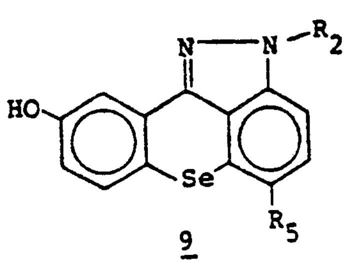

9

from a compound having the structural Formula 6:

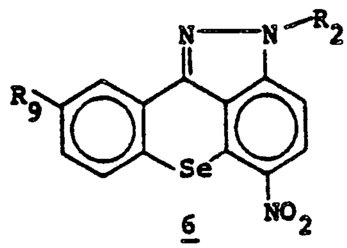

6

wherein $R_2$ has the above meaning, $R_5$ is nitro and $R_9$ is alkoxy of from 1 to 4 carbon atoms and may also be benzyloxy or *p*-halo- or *p*-methoxy-substituted benzyloxy. The conversion is suitably accomplished by hydrolysis with acid such as 48% HBr for compounds wherein $R_9$ is alkoxy or by hydrogenolysis for compounds wherein $R_9$ is benzyloxy.

In still another embodiment, the invention comprises a process for preparing compounds having the structural Formula 10:

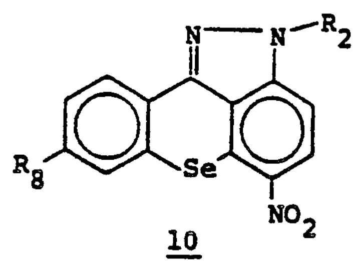

10

by reacting a compound having the structural Formula 14:

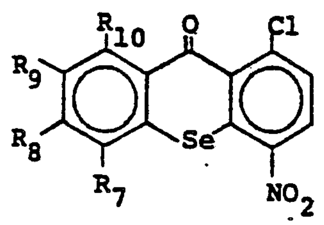

14

with a substituted hydrazine having the structural formula $H_2NNHR_2$; wherein $R_2$ has the above meaning and $R_8$ is hydroxy or alkoxy of from one to four carbon atoms and may also be benzyloxy of halo- or methoxy-substituted benzyloxy.

In still another embodiment, the invention comprises a process for preparing compounds having the structural Formula 11:

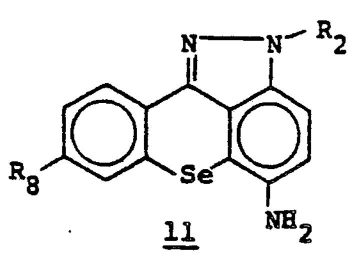

11

by subjecting to reduction a compound having the structural Formula 10:

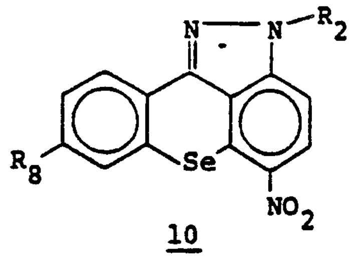

10

wherein $R_2$ has the above meaning and $R_8$ is hydroxy or alkoxy of from 1 to 4 carbon atoms and may also be benzyloxy or halo- or methoxy-substituted benzyloxy. Preferably, the reduction is carried out in AcOH using Pd/C as the catalyst for the methoxy substituted compounds and in MeOH using Raney nickel as the catalyst for the benzyloxy substituted compounds.

The invention also comprises a process for preparing compounds having the structural Formula 12:

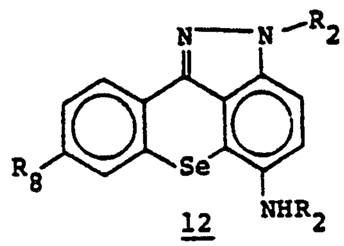

12

by alkylating a compound having the structural Formula 11:

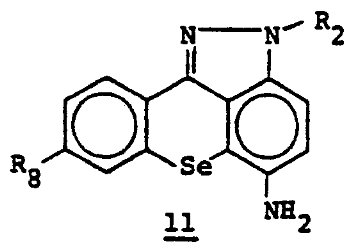

11

wherein $R_2$ has the above meaning and $R_8$ is hydroxy or alkoxy of from 1 to 4 carbon atoms and may also be benzyloxy or *p*-halo- or *p*-methoxy-substituted benzyloxy.

In another embodiment, the invention comprises a process for preparing compounds having the structural Formula 13:

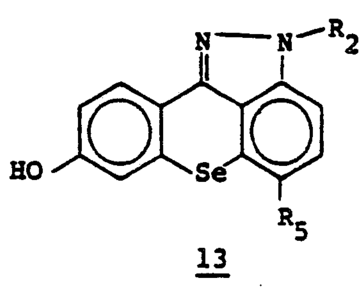

13

from a compound having the structural Formula 10:

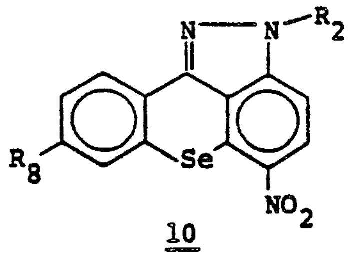

10

wherein $R_2$ has the above meaning, $R_5$ is nitro and $R_8$ is alkoxy of from 1 to 4 carbon atoms and may also be benzyloxy or *p*-halo- or *p*-methoxy-substituted benzyloxy. The conversion is suitably accomplished by hydrolysis with acid such as 48% HBr for compounds wherein $R_8$ is alkoxy or by hydrogenolysis for compounds wherein $R_8$ is benzyloxy.

Purification of compounds or products obtained by the mehtods of the invention is accomplished in any suitable way, preferably by column chromatography or crystallization.

The invention in its composition aspect relates to a pharmaceutical composition comprising a compound having structural Formula 1 and the pharmaceutically acceptable salts thereof in combination with a pharmaceutically acceptable carrier.

The invention in another aspect relates to a pharmaceutical composition comprising a compound having structural Formula 2 and the pharmaceutically acceptable salts thereof in combination with a pharmaceutically acceptable carrier.

The invention in another aspect relates to a pharmaceutical compositions comprising a compound having structural Formula 3 and the pharmaceutically acceptable salts thereof in combination with a pharmaceutically acceptable carrier.

The invention in another pharmaceutical aspect relates to a pharmaceutical composition comprising a compound having structural Formula 7 and the pharmaceutically acceptable salts thereof in combination with a pharmaceutically acceptable carrier.

The invention in another pharmaceutical aspect relates to a pharmaceutical composition comprising a compound having the structural Formula 11

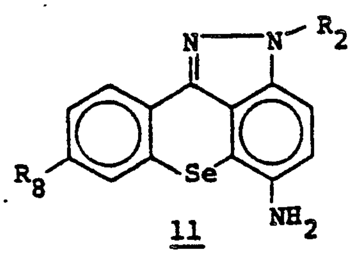

11

and the pharmaceutically acceptable salts thereof in combination with a pharmaceutically acceptable carrier.

The compounds of the present invention having the structural Formula 7 may be used in a pharmaceutical composition for treating solid tumors in a mammal comprising a sufficient amount of a compound having structural Formula 7 and the pharmaceutically acceptable salts thereof in combination with a pharmaceutically acceptable carrier.

The compounds of the present invention having the structural Formula 1 may be used in a pharmaceutical composition for treating microbial infections in a mammal comprising a sufficient amount of a compound having structural Formula 1 and the pharmaceutically acceptable salts thereof in combination with a pharmaceutically acceptable carrier.

The compounds having the structural Formula 1 may be used in a pharmaceutical composition for treating leukemia in a mammal comprising a sufficient amount of compound having structural Formula 1 and the pharmaceutically acceptable salts thereof in combination with a pharmaceutically acceptable carrier.

The compounds having the structural Formula 2 may be used in a pharmaceutical composition for treating leukemia in a mammal comprising a sufficient amount of a compound having structural Formula 2 and the pharmaceutically acceptable salts thereof in combination with a pharmaceutically acceptable carrier.

The compounds having the structural Formula 1 may be used in a pharmaceutical composition for treating solid tumors in a mammal comprising a sufficient amount of a compound having structural Formula 1 and the pharmaceutically acceptable salts thereof in combination with a pharmaceutically acceptable carrier.

The compounds having the structural Formula 2 may be used in a pharmaceutical composition for

treating solid tumors in a mammal comprising a sufficient amount of a compound having structural Formula 2 and the pharmaceutically acceptable salts thereof in combination with a pharmaceutically acceptable carrier.

The compounds having the structural Formula 7 may be used in a pharmaceutical composition for treating solid tumors in a mammal comprising a sufficient amount of a compound having structural Formula 7 and the pharmaceutically acceptable salts thereof in combination with a pharmaceutically acceptable carrier.

## PHYSICAL AND PHARMACOLOGICAL PROPERTIES OF THE COMPOUNDS

The benzoselenino[4,3,2-*cd*]indazole compounds of the invention range in color from beige to yellow. They are generally crystalline solids that are stable under normal atmospheric conditions. The compounds typically have melting points in the range of about 100 to about 250°C.

The compounds are useful as pharmacological agents for the treatment of bacterial and fungal infections in warm-blooded animals. The activity of representative compounds of the invention was established by test protocols described below.

In addition to their usefulness as antibacterial and antifungal agents, compounds of the invention display in vitro and in vivo antitumor activity.

## TEST PROTOCOLS

In Vitro

One test protocol uses L1210 cells, a murine leukemia cell line, grown in RPMI 1640 supplemented with 5% fetal bovine serum and gentamicin (50 µg/ml). Drug dilutions are prepared in the appropriate solvent and 20 µ, of each dilution are added to 24-well Linbro tissue culture plates, followed by the addition of 2.0 ml of cell suspension containing $3 \times 10^4$ cells per ml. Solvent and medium controls are included in each test. After incubation at 37°C for three days in 5% $CO_2$, the contents of each well are removed and the cells counted in a ZBI Coulter counter. Percent growth are calculated relative to the controls and the levels of drug activity are expressed as $ID_{50}$ in moles per liter.

Still another test protocol is the in vitro antibacterial antifungal (ABMF) test. Compounds are tested for antimicrobial activity in a semiautomated broth dilution technique vs a panel of microorganisms. Test organisms include five gram-negative bacteria, seven gram-positive bacteria, four yeasts, and two fungi. The concentrations of evaluated compounds range from 0.46—1000 µg/ml.

Broth dilution tests are considered to be "the most quantitative method for antimicrobial susceptibility testing" (Sherris and Washington, in Lennette, E. H., ed., Manual of Clincial Microbiology, Washington. American Society for Microbiology, pp 446—452, 1980.)

Dilution tests are performed by making serial dilutions of a test agent in broth, inoculating with a given test organism, then incubating the inoculated media. The minimal inhibitory concentration (MIC) is determined as the lowest concentration of compound yielding no visible growth of the test organism.

For convenience, values are reported for three gram-negative bacteria (*Salmonella typhimurium, Escherichia coli,* and *Branhamella catarrhalis*) and two gram-positive bacteria (*Streptococcus pneumoniae* and *Streptococcus faecalis*).

In Vivo

Another test protocol is the in vivo lymphocytic leukemia P388 test. The animals used are either male or female $CD_2F_1$ mice. There are six or seven animals per test group. The tumor transplant is by intraperitoneal injection of dilute ascitic fluid containing cells of lymphocytic leukemia P388. The test compounds are administered intraperitoneally once daily for five consecutive days at various doses following tumor inoculation. The animals are weighed and survivors are recorded on a regular basis for 30 days. A compound is designated "toxic" if, at a given dose, all animals died prior to four days after the first injection of drug. A ratio of survival time for treated (T)/control (C) animals is calculated. A criterion for efficacy is a ratio T/C times 100 greater than or equal to 125. See *Cancer Chemotherapy Reports,* Part 3, *3*; 1 (1972), for a comprehensive discussion of the protocol.

These test protocol procedures gave results listed in Table 1 for representative compounds of the invention.

TABLE 1

Chemical, Antitumor, Antibacterial, and Antifungal Data on 2,5-(disubstituted)-2H-[1]benzoselenino[4,3,2-cd]indazoles

| $R_2$ | $R_5$ | $R_7$—$R_{10}$ | Formula | mp °C | L1210 in vitro $ID_{50}$ (M) | P388 in vivo Dose (mg/kg) | P388 in vivo T/C × 100 | MIC (μg/ml) S.Typhimurium | MIC E. Coli | MIC S. Pneumoniae | MIC S. Paecalis | MIC B. Catarrhalis |
|---|---|---|---|---|---|---|---|---|---|---|---|---|
| $CH_2CH_2Net_2$ | $NO_2$ | H | $C_{19}H_{20}N_4O_2Se \cdot 0.9\ HCl \cdot 0.4\ H_2O$ | 273—274 dec | $1.4 \times 10^{-7}$ | | | | | | | |
| $CH_2CH_2N(CH_2)_4$ | $NO_2$ | H | $C_{19}H_{18}N_4O_2Se \cdot 0.8\ HCl \cdot 1.2\ H_2O$ | | | | | | | | | |
| $CH_2CH_2NEt_2$ | $NO_2$ | 9—OH | $C_{19}H_{20}N_4O_3Se \cdot 0.5\ HCl \cdot 0.7\ H_2O$ | 247—249 dec | $4.1 \times 10^{-9}$ | | | 0.46 | 0.46 | 0.46 | 0.46 | 0.46 |
| $CH_2CH_2NEt_2$ | $NO_2$ | 8—OH | $C_{19}H_{20}N_4O_3Se \cdot 1.0\ HCl$ | 248—252 dec | $2.0 \times 10^{-7}$ | | | | | | | |
| $CH_2CH_2NEt_2$ | $NH_2$ | H | $C_{19}H_{22}N_4Se \cdot 2.0\ HCl$ | 262—264 | $1.3 \times 10^{-7}$ | | | 111 | 37 | 12.3 | 37 | 12.3 |
| $CH_2CH_2N(CH_2)_4$ | $NH_2$ | H | $C_{19}H_{20}N_4Se \cdot 1.94\ HCl \cdot 0.52\ H_2O$ | >300 | | | | 37.0 | 12.3 | 4.1 | 37 | 12.3 |
| $CH_2CH_2NEt_2$ | $NHCH_2CH_2NH_2$ | H | $C_{21}H_{27}N_5Se \cdot 2.8\ HCl \cdot 1.4\ H_2O$ | 255—258 dec | $3.3 \times 10^{-8}$ | 12.50<br>6.25 | 270 (cures)<br>205 | 12.3 | 12.3 | 4.1 | 12.3 | 12.3 |
| $CH_2CH_2NEt_2$ | $NHCH_2CH_2NEt_2$ | H | $C_{25}H_{35}N_5Se \cdot 2.0\ HBr \cdot 0.2\ H_2O$ | 243—244 | $2.5 \times 10^{-7}$ | 50.00<br>25.00 | 174<br>127 | 111.0 | 111.0 | 4.1 | 111.0 | 111.0 |
| $CH_2CH_2NEt_2$ | $NHCH_2CH_2CH_2NH_2$ | H | $C_{22}H_{29}N_5Se \cdot 2.9\ HBr \cdot 1.9\ H_2O$ | 255—256 | $1.6 \times 10^{-7}$ | | | 37.0 | 111.0 | 4.1 | 111.0 | 37.0 |
| $CH_2CH_2N(CH_2)_4$ | $NHCH_2CH_2NH_2$ | H | $C_{21}H_{25}N_5Se \cdot 2.7\ HCl \cdot 0.6\ H_2O$ | 265—272 dec | $5.5 \times 10^{-8}$ | | | 12.3 | 12.3 | 1.4 | 12.3 | 4.1 |
| $CH_2CH_2NEt_2$ | $NHCH_2CH_2NH_2$ | 9—OH | $C_{21}H_{27}N_5OSe \cdot 3.0\ HCl \cdot 0.3\ H_2O$ | >260 dec | $2.4 \times 10^{-9}$ | 3.12 | 198 | 37.0 | 1.4 | 4.1 | 37.0 | 4.0 |

PREPARATION OF PHARMACEUTICAL COMPOSITIONS

When being utilized as antibiotic and antifungal agents, the compounds of the invention can be prepared and administered in a wide variety of topical, oral, and parenteral dosage forms. It will be clear to those skilled in the art that the following dosage forms may comprise as the active component, one or more compounds of Formula 1, a corresponding pharmaceutically acceptable salt of any of said compounds, or a mixture of such compounds and/or salts.

For preparing pharmaceutical compositions from the compounds described by this invention, inert, pharmaceutically acceptable carriers can be either solid or liquid. Solid form preparations include powders, tablets, dispersible granules, capsules, catchets, and suppositories. A solid carrier can be one or more substances which may also act as diluents, flavoring agents, solubilizers, lubricants, suspending agents, binders, or tablet disintegrating agents; it can also be an encapsulating material. In powders the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, and cocoa butter. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspensions suitable for oral use can be made by dispersing the finely divided active component in water with viscous material, i.e., natural or synthetic gums, resins, methyl cellulose, sodium carboxymethyl cellulose, and other well-known suspending agents.

Topical preparations include dusting powders, creams, lotions, gels, and sprays. These various topical preparations may be formulated by well-known procedures. See for example Remington's Pharmaceutical Sciences, Chapter 43, 14th Ed., 1970, Mack Publishing Co., Easton, Pennsylvania 18042, USA.

Preferably, the pharmaceutical preparation is in unit dosage form. In such form, the preparation is subdivided into unit doses containing appropriate quantities of the active component. The unit dosage form can be packaged preparation, the package containing discrete quantities of preparation, for example, packeted tablets, capsules, and powders in vials or ampoules. The unit dosage form can also be a capsule, cachet, or tablet itself or it can be the appropriate number of any of these packaged forms.

The quantity of active compound in a unit dose of preparation may be varied or adjusted from 50 mg to 500 mg according to the particular application and the potency of the active ingredient.

In therapeutic use as antibiotic and antifungal agents, the compounds utilized in the pharmaceutical method of this invention are administered at the initial dosage of about 0.1 mg to about 50 mg per kilogram. A dose range of about 0.5 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaraneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), N,N-dimethylacetamide, suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid or thimrosal. In many cases, it will be preferable to include isotonic agents, for example, sugars or sodium chloride. Prolonged absorption of the injectable compositions can be brought about by the use in the compositions of agents delaying absorption, for

example, aluminum monostearate and gelatin.

Sterile injectable solutions are prepared by incorporating the active compound in the required amount in the appropriate solvent with various of the other ingredients enumerated above, as required, followed by sterilization accomplished by filtering. Generally, dispersions are prepared by incorporating the various sterilized active ingredient into a sterile vehicle which contains the basic dispersion medium and the required other ingredients from those enumerated above. In the case of the sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutically active substances is well-known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in unit dosage form for ease of administration and uniformity of dosage. Unit dosage form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel unit dosage forms of the invention are dictated by and directly dependent on (a) the unique characteristics of the active material and the particular therapeutic effect to be achieved, and (b) the limitation inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in unit dosage form as hereinbefore disclosed. A unit dosage form can, for example, contain the principal active compound in amounts ranging from about 0.1 to about 500 mg, with from about 0.5 to about 250 mg being preferred. Expressed in proportions, the active compound is generally present in from about 0.1 to about 500 mg/ml of carrier. In the case of compositions containing supplementary active ingredients, the dosages are determined by reference to the usual dose and and the manner of administration of the said ingredients. The daily parenteral doses for mammalian subjects to be treated ranges from 0.1 mg/kg to 100 mg/kg. The preferred daily dosage range is 0.3 mg/kg to 10 mg/kg.

The invention and the best mode of practicing the same are illustrated by the following examples of preferred embodiments of selected compounds and their preparations.

PREPARATION OF STARTING MATERIALS

Example 1

1-Chloro-4-nitro-9*H*-seloxanthen-9-one

A stirred solution of 32 g of phosphorus pentoxide and 320 g of methanesulfonic acid at 80°C is treated with 16 g (0.045 mole) of 2-[(5-chloro-2-nitrophenyl)seleno]benzoic acid. The mixture is stirred at 80°C for two hours, cooled to 25°C, and poured into 900 ml of chilled water. The suspension is stirred for 30 minutes and the solids are collected by filtration, then washed sequentially with water, 5% aqueous sodium bicarbonate, and 2-propanol to give 6.2 g of the dried product; mp 189—190°C.

Alternatively, 1-chloro-4-nitro-9*H*-selenoxanthen-9-one may be prepared as follows.

A suspension of 140 g of phosphorus pentoxide in 700 ml of dichloromethane is refluxed with 350 ml of hexamethyldisiloxane for two hours. The solution is concentrated in vacuo to a clear colorless syrup.

A mixture of 125 g of the syrup and 15.5 g of additional phosphorus pentoxide is heated at 210°C. To this mechanically stirred suspension is added 4.75 g (0.016 mole) of 2-[(5-chloro-2-nitrophenylseleno]-benzoic acid. After 15 minutes, the reaction is cooled to 25°C and poured into ice cold 6 N aqueous hydrochloric acid. After stirring for one hour, the gold solid is collected and dried in vacuo at 70°C for 13 hours to give 4.47 g of dried product; mp 196—197°C.

2-[(5-Chloro-2-nitrophenyl)seleno]benzoic acid is prepared as follows:

A stirred mixture of 20 g (0.088 mole) of 2-selenocyanobenzoic acid and 300 ml of ethanol under argon at 0°C is treated portionwise with 13.5 g (0.36 mole) of sodium borohydride. The mixture is stirred at 25°C for 40 minutes and treated dropwise with a solution of 17 g (0.089 mole) of 2,4-dichloronitrobenzene in 50 ml of ethanol. The mixture is heated under reflux for 15 hours, cooled to 0°C, treated with 1.7 g (0.045 mole) of additional sodium borohydride, and refluxed another two hours. The cooled mixture is acidified with one normal aqueous hydrochloric acid, then extracted with ethyl acetate. The combined extracts are concentrated to a solid residue whose crystallization from acetonitrile gives 26.3 g of product; mp 226—228°C.

2-Selenocyanobenzoic acid is prepared as follows:

A mixture of 54.7 g (0.25 mole) of 2-bromobenzoic acid, sodium salt, 105 ml of a 2.36 molar solution of sodium cyanoselenate in *N,N*-dimethylacetamide [*J. Org. Chem.*, *43*, 1689 (1978)], 200 mg of copper

powder, and 50 ml of *N,N*-dimethylacetamide is heated under nitrogen at 150°C for four hours. The hot solution is poured into 450 ml of ice water. The aqueous mixture is acidified with 30 ml of concentrated hydrochloric acid then extracted three times with 100 ml portions of dichloromethane. The combined extracts are clarified with charcoal then stirred for four hours under nitrogen with a solution of 28 g of sodium bicarbonate in 300 ml of water. The aqueous layer is clarified with charcoal, cooled, and acidified with 28 ml of concentrated hydrochloric acid. The solids are collected, washed with ice-cold water, and dried to give 38.6 g of the product; mp 170—174°C.

Example 2

1-Chloro-7-methoxy-4-nitro-9*H*-selenoxanthen-9-one

A mixture of 200 g of the hexamethyldisiloxane/phosphorus pentoxide syrup and 24 g of additional phosphorus pentoxide is heated to 210°C. To this mechanically stirred suspension is added 6.0 g (0.015 mole) of 2-[(5-chloro-2-nitrophenyl)seleno]-5-methoxybenzoic acid, hydrochloride. After 20 minutes, the reaction is cooled to 25°C and poured over ice. After stirring for three hours, the orange solid is collected on the Celite® pad. The product with the Celite® pad is stirred in ethyl acetate at 25°C for 13 hours. The mixture is filtered and the Celite® washed with hot ethyl acetate The filtrate is concentrated to a bright orange solid whose recrystallization from acetonitrile gives 3.58 g of dried product; mp 222—224°C.

2-[(5-Chloro-2-nitrophenyl)seleno]-5-methoxy benzoic acid, hydrochloride is prepared as follows:

A stirred mixture of 7.68 g (0.030 mole) of 2-selenocyano-5-methoxybenzoic acid and 6.90 g (0.036 mole) of 2,4-dichloronitrobenzene in 290 ml of ethanol under an argon atmosphere at 0°C is treated portionwise with 3.42 g (0.090 mole) of sodium borohydride. After the addition is complete, the reaction is warmed to 25°C. The mixture is heated under reflux for 18 hours, cooled, acidified with 1 N aqueous hydrochloric acid, and then extacted into ethyl acetate. The extracts are concentrated to 9.8 g of analytically pure product as a salt with 0.10 equivalents of hydrogen chloride and solvated with 0.13 equivalents of water; mp 194—196°C.

2-Selenocyano-5-methoxybenzoic acid is prepared as follows:

A mixture of 15.0 g (0.059 mole) of 2-bromo-5-methoxybenzoic acid, (*J. Amer. Chem. Soc. 68,* 1599 (1946]) sodium salt, 225 ml of *N,N*-dimethylacetamide, 11.10 g (0.077 mole) of potasium selenocyanate, and 0.75 g of copper powder is heated under argon at 150°C for four hours. The reaction is cooled, poured onto crushed ice, and acidified with 25 ml of concentrated hydrochloric acid. The precipitate is collected and slurried with a 5 to 1 (v/v) mixture of dichloromethane and tetrahydrofuran. The mixture is stirred overnight with charcoal. Filtration through a Celite® pad gives a pale yellow filtrate to which is added a solution of 14 g of sodium bicarbonate in 150 ml of water. After stirring under an argon atmosphere for several hours, the organic layer is separated and extracted once with saturated aqueous sodium bicarbonate solution. The combined aqueous solutions are cooled with crushed ice and acidified with concentrated hydrochloric acid. The solids are collected, washed with cold water, and dried to give 7.71 g of the product; mp 183—186°C (dec.).

Example 3

1-Chloro-6-hydroxy-4-nitro-9*H*-selenoxanthen-9-one

Aluminum chloride (anhydrous powder, 2.34 g) is added to a suspension of 2.16 g (0.006 mole) 1-chloro-6-methoxy-4-nitro-9*H*-selenoxanthen-9-one in 25 ml of 1,2-dichloroethane. The mixture is heated at 75°C for 1.5 hours, then concentrated in vacuo. The residue is treated with 50 ml of concentrated hydrochloric acid at 25°C for six hours. The solids are collected, washed with water, and dried in vacuo to give 1.73 g of product solvated with 0.74 equivalents of water. The product so obtained is sufficiently pure for further reaction.

1-Chloro-6-methoxy-4-nitro-9*H*-selenoxanthen-9-one, mp 229—232°C (dec) is prepared in a manner analogous to that described for the preparation of 1-chloro-7-methoxy-4-nitro-9*H*-selenoxanthen-9-one. The requisite 2-[(5-chloro-2-nitrophenyl)seleno-4-methoxy benzoic acid is similarly obtained by coupling 2-selenocyano 4-methoxybenzoic acid and 2,4-dichloronitrobenzene. By treating 30.0 g of 2-iodo-4-methoxybenzoic acid [*Coll. Czech, Chem. Com., 39* 3548 (1974)] with potassium selocyanate there is obtained 21.13 g of 2-selenocyano-4-methoxybenzoic acid, mp 225—228°C (decarboxylation).

1-(2-Hydrozinoethyl)pyrrolidine

A mixture of 200 g of 85% hydrazine hydrate, 200 ml of water, 170 g of *N*-chloroethylpyrrolidine hydrochloride, and 70 g of potassium carbonate is boiled under reflux for seven hours. Sodium hydroxide (390 g) is added and the mixture is extracted with ether. The ethereal extract, dried and distilled, yields the title compound, bp 107—111°C (18.5 mm Hg).

Example 4

*N,N*-Diethyl-5-nitro-2*H*-[1]benzoselenino[4,3,2-*cd*]indazole-2-ethanamine

An ice-cold solution of 6.2 g (0.018 mole) of 1-chloro-4-nitro-9*H*-selenoxanthen-9-one, 90 ml of *N,N*-dimethylformamide and 9.6 ml of diisopropyl ethylamine is treated dropwise during five minutes with 3.3 g (0.032 mole) of 2-diethylaminoethyl)hydrazine [*J. Med. Chem., 7,* 493 (1964)]. The cooling bath is removed after ten minutes and the mixture is stirred at 25°C for 30 minutes. Concentration of the mixture in vacuo

leaves an orange solid that is triturated with hot 2-propanol to give 7.0 g of the dried product as a salt with 0.7 equivalents of hydrogen chloride and solvated with 0.2 equivalent of water; mp 246—248°C.

Example 5

5-Amino-*N,N*-diethyl-2*H*-[1]benzoselenino[4,3,2-*cd*]indazole-2-ethanamine

A mixture of 1.5 g (0.003 mole) of *N,N*-diethyl-5-nitro-2*H*[1]benzoselenino[4,3,2-*cd*]indazole-2-ethanamine, hydrochloride, 60 mg of 20% palladium on carbon, and 9 ml of acetic acid is hydrogenated at atmospheric pressure for 18 hours. The mixture is filtered and concentrated in vacuo to give a solid residue. Dissolution in a solution of hydrogen chloride in 2-propanol gives 1.4 g of the dried product as a salt with 2.0 equivalents of hydrogen chloride and solvated with 0.4 equivalent of water and 0.6 equivalent of 2-propanol; mp 262—264°C (dec.).

Example 6

*N*-[2-[2-(diethylamino)ethyl]-2*H*-[1]-benzoselenino[4,3,2-*cd*]indazol-5-yl]-1,2-ethanediamine

A mixture of 3.7 g (0.01 mole) of 5-amino-*N,N-diethyl-2H*[1]benzoselenino[4,3,2-*cd*]indazole-2-ethanamine, 5.9 g (0.029 mole) of 2-bromoethylamine, hydrobromide, and 25 ml of ethanol is heated under reflux for ten hours. Additional 2-bromoethylamine, hydrobromide (2.0 g) is added and the solution is heated at reflux for 38 hours, then allowed to cool to 25°C. The solids are collected and washed with cold ethanol to give 3.5 g of the dried product as a salt with 2.8 equivalents of hydrogen bromide; mp 268—270°C (dec.).

Example 7

*N,N*-diethyl-5-[[2-(diethylamino)ethyl]amino]-2*H*-[1]-benzoselenino[4,3,2-*cd*]indazole-2-ethanamine

A mixture of 1.47 g (0.004 mole) of 5-amino-*N,N*-diethyl-2*H*-[1]-benzoselenino[4,3,2-*cd*]indazol-2-ethanamine, 3.0 g (0.02 mole) 2-diethylaminoethylbromide, hydrobromide, and 25 ml absolute ethanol is heated under reflux for 24 hours. Additional 2-diethylaminoethyl bromide, hydrobromide (3.0 g) is added portionwise during the next 64 hours. After 88 hours, the reaction is cooled to 0°C. The solid is collected and washed with cold ethanol to give 1.20 g of the dried crude product. Recrystallization from ethanol/2-propanol (3/1) gives 1.1 grams of dried product as a salt with 1.98 equivalents of hydrogen bromide and solvated with 0.06 equivalent of water; mp 243—244°C (dec.).

Example 8

*N*-[2-[2-(diethylamino)ethyl]-2*H*-[1]-benzoselenino[4,3,2-*cd*]indazol-5-yl]-1,3-propanediamine

A mixture of 3.08 g (.008 mole) of 5-amino-*N,N*-diethyl-2*H*[1]benzoselenino[4,3,2-*cd*]indazole-2-ethanamine, 5.26 g (0.02 mole) of 3-bromopropylamine, hydrobromide, and 50 ml of absolute ethanol is heated under reflux for 24 hours. Additional 3-bromopropylamine, hdyrobromide, (4.5 g) is added portionwise during the next 40 hours. After 64 hours, the reaction is cooled to 0°C. The solid is collected and washed with cold ethanol to give 1.92 g of the dried crude product. Recrystallization twice from ethanol/methanol (1/1) gives 0.375 g of the dried product as a salt with 2.91 equivalents of hydrogen bromide and solvated with 1.91 equivalents of water that is 87% pure according to high performance liquid chromatography; mp 255—256°C (dec.).

Example 9

5-Nitro-2-[1-(1-pyrrolidinyl)ethyl])2*H*-[1]benzoselenino[4,3,2-*cd*]indazole, hydrochloride

An ice cold solution of 4.47 g (0.013 mole) of 1-chloro-4-nitro-9*H*-selenoxanthen-9-one in 50 ml of *N,N*-dimethyl-formamide with 7 ml of diisopropylethylamine is treated dropwise with 3.4 g of 1-(2-hydrazino-ethyl)pyrrolidine. The cooling bath is removed after 10 minutes and the reaction is stirred at 25°C for one hour. Concentration of the reaction in vacuo leaves an orange solid that is triturated with boiling 2-propanol to give 4.78 g of the proudct as a salt with 1.18 equivalents of water and 0.81 equivalents of hydrogen chloride, mp 258—262°C.

Example 10

5-Amino-2-[2-(1-pyrrolidinyl)ethyl]-2*H*-[1]benzoselenino[4,3,2-*cd*]indazole, hydrochloride

A suspension of 3.09 g (0.006 mole) of 5-nitro-2-[2-(1-pyrrolidinyl)ethyl])2*H*-[1]benzoselenino[4,3,2-*cd*]-indazole, hydrochloride and 0.250 g of 20% palladium on carbon in 100 ml of methanol and 20 ml of glacial acetic acid is hydrogenated at atmospheric pressure for 13 hours. The mixture is filtered through Celite® and concentrated in vacuo to an orange solid. The solid is evaporated several times from n-heptane to remove residual acetic acid. The resulting solid is dissolved in warm 2-propanol and acidified with a solution of hydrogen chloride in 2-propanol to give a fluffy off white precipitate. The solid is collected and dried in vacuo to give 2.99 g of the product as a salt with 1.94 equivalents of hydrogen chloride and solvated with 0.52 equivalents of water; mp > 300°C.

Example 11

5-[(2-Aminoethyl)amino]-2-[2-(1-[pyrrolidinyl)ethyl)-2*H*-[1]benzoselenino[4,3,2-*cd*]indazol, hydrochloride

A suspension of 2.10 g (0.003 mole) of 2-[2-[[[2-(1-pyrrolidinyl)ethyl]-2*H*-[1]benzoselenino[4,3,2-*cd*]-indazol-5-yl]amino]ethyl]-1-*H*-isoindole-1,3(2*H*)-dione in 80 ml of methanol with 4.5 ml of methylhydrazine

is heated under reflux in an argon atmosphere for 17 hours. The solution is concentrated in vacuo several times from 2-propanol and the residue is dissolved in a 1 to 1 mixture of methanol and 2-propanol. To this solution is added 5 ml of a solution of hydrogen chloride in 2-propanol (1.22 g of hydrogen chloride in 10 ml of 2-propanol). The resulting solid is triturated with boiling 2-propanol to give 1.34 g of the product as a salt with 2.74 equivalents of hydrogen chloride and solvated with 0.61 equivalents of water and 0.14 equivalents of 2-propanol; mp 265—272°C (decomposition).

2 - [2 - [[[2 - (1 - Pyrrolidinyl)ethyl] - 2*H* - [1]benzoselenino[4,3,2 - *cd*]indazol - 5 - yl]amino]ethyl] - 1 - *H*-isoindole - 1,3(2*H*) - dione is prepared as follows:

5-Amino-2-[2-(1-pyrrolidinyl)ethyl]-2*H*-[1]benzoselenino[4,3,2-*cd*]indazole is added to (2.39 g, 0.009 mole) of N-(2-bromoethyl]phthalimide and the mixture is heated under an argon atmosphere at 110°C for 24 hours. An additional 1.2 g portion of N-(2-bromoethyl)phthalimide is added and heating is continued for another six hours. The reaction is cooled to room temperature and dichloromethane is added with 2.5 ml of diisopropylethylamine. The mass is heated under reflux for ten hours under argon. After cooling to 25°C, the reaction is diluted with ethyl acetate and washed twice with a solution of saturated aqueous sodium bicarbonate. The organic portion is separated, dried over sodium sulfate, filtered, and concentrated to a dark oil. chromatography on silica gel with a solution of 5% dichloromethane gives 2.10 g of product as a foamy solid sufficiently pure for further reaction.

Example 12

2-[2-(Diethylamino)ethyl]-5-nitro-2*H*-[1]benzoselenino[4,3,2-*cd*]indazol-9-ol, hydrochloride

1-Chloro-7-methoxy-4-nitro-9*H*-selenoxanthen-9-one (3.49 g, 0.010 mole) is suspended in 50 ml of dry 1,2-dichloroethane. To this mixture is added 3.77 g (0.028 mole) of powdered aluminum chloride and the suspension is heated under reflux in an argon atmosphere for 2.5 hours. The reaction is cooled to room temperature and evaporated in vacuo to a dark residue. The residue is stirred with 100 ml of concentrated hydrochloric acid under argon for 15 hours. The solids are collected, rinsed with cold water, triturated with warm 2-propanol, and dried in vacuo at 70°C to give 4.74 g of crude product. An ice cold solution of 0.536 g (0.015 mole) of crude phenol in 5 ml of *N,N*-dimethylformamide with 0.525 ml of diisopropylethylamine is treated dropwise with 0.350 ml of 2-(diethylaminoethyl)hydrazine. The cooling bath is removed after ten minutes and the mixture is stirred at 25°C for two hours. Concentration of the reaction in vacuo leaves an orage solid that is triturated with boiling 2-propanol to give 0.527 g of the dried product as a salt with 0.45 equivalents of hydrogen chloride and solvated with 0.74 equivalents of water; mp 247—249°C (decomposition).

Example 13

2-[2-[Diethylamino)ethyl]-5-nitro-2*H*-[1]benzoselenino[4,3,2-*cd*]indazol-8-ol, hydrochloride

To an ice-cold solution of 1.59 g (0.004 mole) of 1-chloro-6-hydroxy-4-nitro-9*H*-selenoxanthen-9-one in 12 ml of N,N-dimethylformamide with 0.85 ml of diisopropylethyl amine is added 0.85 ml of 2-(diethylaminoethyl)hydrazine. The cooling bath is removed and the mixture is stirred at 25°C for 2.5 hours. Concentration of the reaction in vacuo leaves an orange solid. Trituration of the solid with boiling 2-propanol gives an orange powder which is dried in vacuo at 80°C to afford 1.66 g of the dry product as a salt with 1.0 equivalents of hydrogen chloride; mp 255—257°C (dec.).

Example 14

5-[(2-Aminoethyl)amino]-2-[2-(diethylamino)ethyl]-2*H*-[1]benzoselenino[4,3,2-*cd*]indazol-9-ol, trihydrochloride

A suspension of 1.95 g (0.003 mole) of 2 - [2 - [[[2 - (diethylamino)ethyl] - 9 - hydroxy - 2*H* - [1]-benzoselenino[4,3,2 - *cd*]indazol - 5 - yl]amino]ethyl] - 1 - *H* - isoindole - 1,3(2*H* - dione in 80 ml of methanol with 4.5 ml of methylhydrazine is heated under reflux in an argon atmosphere for 18 hours. The solution is concentrated in vacuo several times from 2-propanol and the residue is dissolved in a 1:1 (v/v) mixture of methanol and 2-propanol. To this solution is added 4 ml of a solution of hydrogen chloride in 2-propanol (1.22 g of hydrogen chloride in 10 ml of 2-propanol). The pale yellow solid is collected and recrystallized from methanol and 2-propanol to give 1.724 g of the dry product as a salt with three equivalents of hydrogen chloride and solvated with 0.30 equivalents of water; mp >260°C.

2 - [2 - [[[2 - (Diethylamino)ethyl] - 9 - hydroxy - 2H - [1]benzoselenino[4,3,2 - *cd*]indazol - 5 - yl]-amino]ethyl] - 1 - *H* - isoindole - 1,3(2*H*)dione is prepared as follows:

A suspension of 4.28 g (0.009 mole) of 2-[2-(diethylamino)ethyl]-5-nitro-2*H*-[1]benzoselenino[4,3,2-*cd*]-indazol-9-ol, hydrochloride and 0.650 g of 20% palladium on carbon in 100 ml *N,N*-dimethylformamide is hydrogenated at atmospheric pressure for 13 hours. An additional 0.25 g of catalyst is added and the hydrogenation continues for another 24 hours. The mixture is filtered through a Celite® pad and the filtrate is concentrated in vacuo to 4.74 g of a dark orange-brown oil. To this oil is added 2.67 g (0.011 mole) of N-(2-bromoethyl)phthalimide and the mixture is heated under argon in a 110°C oil bath for 20 hours. To the resulting solid is added 200 ml of 1,2-dichloroethane and 3.3 ml of diisopropylethylamine. The mixture is heated under reflux in an argon atmosphere for two hours, diluted further with dichloromethane, and washed once with a saturated solution of aqueous sodium bicarbonate. The organic layer is separated, dried over sodium sulfate, filtered, and concentrated to a crude solid. The solid is dissolved in

dichloromethane and filtered through a small volume of Florisil with a 1:9 (v/v) solution of methanol and 1,2-dichloroethane. The effluent is concentrated and the resulting solid is triturated with hot 2-propanol to give 1.98 g of the dried product; mp 203—205°C.

Starting from the appropriately substituted 7,8,9 or 10-hydroxy or methoxy 1-chloro-4-nitro-9*H*-selenoxanthen-9-ones, the following 2-(aminoalkyl)-5-(aminoalkylamino)-2*H*-[1]benzoselenino[4,3,2-*cd*]indazoles are prepared by methods similar to those detailed in Example 4:

5-[[2-aminoethyl)amino]-2-[2-(diethylamino)ethyl]-2H-[1]benzoselenino[4,3,2-*cd*]indazol-8-ol;

2-[2-(diethylamino)ethyl]-5-[[2-[(2-hydroxyethyl)amino]ethyl]amino]-2*H*-[1]benzoselenino[4,3,2-*cd*]-indazol-8-ol;

2-[2-[(2-hydroxyethyl)amino]ethyl]-5-[[2-[(2-hydroxyethyl)amino]ethyl]amino-2*H*-[1]benzoselenino-[4,3,2-*cd*]indazol-8-ol;

5-[(2-aminoethyl)amino]-2-[2-(diethylamino)ethyl]-2*H*-[1]benzoselenino[4,3,2-*cd*]indazol-9-ol;

2-[2-(diethylamino)ethyl]-5-[[2-[(2-hydroxyethyl)amino]ethyl]amino]-2*H*-[1]benzoselenino[4,3,2-*cd*]indazol-9-ol;

2-[2-](2-hydroxyethyl)amino]ethyl]-5-[[2-[(2-hydroxyethyl)amino]ethyl]amino]-2*H*-[1]benzoselenino-[4,3,2-*cd*]indazol-9-ol;

5-[(3-aminopropyl)amino]-2-[2-(diethylamino)ethyl]-2*H*-[1]benzoselenino[4,3,2-*cd*]indazol-9-ol;

5-[(aminoethyl)amino]-2-[2-(diethylamino)ethyl]-2*H*-[1]benzoselenino[4,3,2-*cd*]indazol-10-ol;

2-[2-(diethylamino)ethyl]-5-[[2-[(2-hydroxyethyl)amino]ethyl]amino]-2*H*-[1]benzoselenino[4,3,2-*cd*]-indazol-10-ol;

2-[2-[(2-hydroxyethyl)amino]ethyl]-5-[[2-(2-hydroxyethyl)amino]ethyl]amino]-2*H*-[1]benzoselenino-[4,3,2-*cd*]indazol-10-ol;

5-[(2-aminoethyl)amino]-2-[2-(diethylamino)ethyl]-2*H*-[1]benzoselenino[4,3,2-*cd*]indazole-7,8-diol;

2-[2-(diethylamino)ethyl]-5-[[2-[(2-hydroxyethyl)amino]ethyl]amino]-2*H*-[1]benzoselenino[4,3,2-*cd*]-indazole-7,8-diol;

2-[2-[(2-hydroxyethyl)amino]ethyl]-5-[[2-[(2-hydroxyethyl)amino]ethyl]amino]-2*H*-[1]benzoselenino-[4,3,2-*cd*]indazole-7,8-diol;

5-[(2-aminoethyl)amino]-2-[2-(diethylamino)ethyl]-2*H*-[1]benzoselenino[4,3,2-*cd*]indazole-8,10-diol;

2-[2-(diethylamino)ethyl]-5-[[2-[(2-hydroxyethyl)amino]ethyl]amino]-2*H*-[1]benzoselenino[4,3,2-*cd*]-indazole-8,10-diol; and

2-[2-[(2-hydroxyethyl)amino]ethyl]-5-[[2-[(2-hydroxyethyl)amino]ethyl]amino]-2*H*-[1]benzoselenino-[4,3,2-*cd*]indazole-8,10-diol.

**Claims for the Contracting States: BE CH DE FR GB IT LI LU NL SE**

1. A benzoselenino[4,3,2-cd]indazole compound having in free base form the following general formula I:

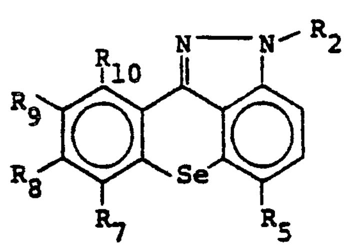

(I)

and the pharmaceutically acceptable salts thereof, wherein:

$R_7$, $R_8$, $R_9$ and $R_{10}$ independently represent hydrogen, hydroxy or alkoxy of from one to four carbon atoms;

$R_2$ is ANR'R'' in which A is a straight or branched alkylene chain of from two to five carbon atoms, optionally substituted with hydroxyl and in which R' and R'' are independently a hydrogen, a straight or branched alkyl of from one to four carbon atoms, optionally substituted with hydroxyl or R' and R'' when taken together, represent the bifunctional radical

$$\begin{matrix} -(CH_2)_n & \\ & \diagdown \\ & B \\ & \diagup \\ -(CH_2)_m & \end{matrix}$$

in which n and m are each, independently, the integer 2 or 3 and B is a direct bond or O, S, or NR''' wherein

R''' is hydrogen or straight or branched alkyl of from one to four carbon atoms, optionally substituted with hydroxyl; and

$R_5$ is nitro, $NH_2$, $NHR_2$ or NHR'''' wherein R'''' is a one to four carbon atom acyl radical, a chloroacetyl radical,

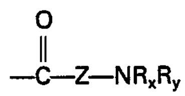

wherein Z is a straight or branched alkylene chain of from one to four carbon atoms and wherein $R_x$ and $R_y$ are each hydrogen, or a straight or branched alkyl of from one to four carbon atoms optionally substituted with hydroxy, or R'''' is the group

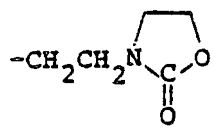

2. A benzoselenino[4,3,2-*cd*]indazole compound according to Claim 1, having in free base form the following general formula:

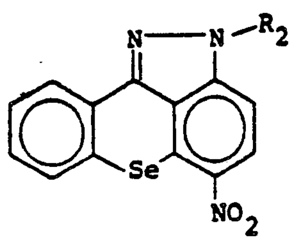

and the pharmaceutically acceptable salts thereof, wherein $R_2$ is as defined in Claim 1.

3. A benzoselenino[4,3,2-*cd*]indazole compound according to Claim 1, having in free base form the following general formula:

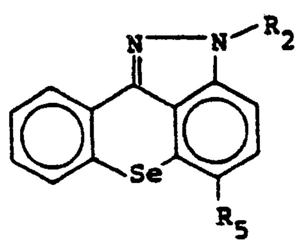

and the pharmaceutically acceptable salts thereof, wherein $R_2$ and $R_5$ are as defined in Claim 1.

4. A compound according to Claim 3, wherein $R_2$ is diethylaminoethyl and $R_5$ is aminoethylamino.

5. A benzoselenino[4,3,2-*cd*]indazole compound according to Claim 1, having in free base form the following general formula:

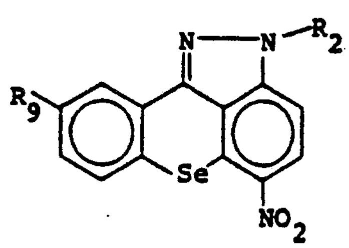

and the pharmaceutically acceptable salts thereof wherein

$R_9$ is hydroxy or alkoxy of from one to four carbon atoms; and

$R_2$ is as defined in Claim 1.

6. A benzoselenino[4,3,2-*cd*]indazole compound according to Claim 1, having in free base form the following general formula:

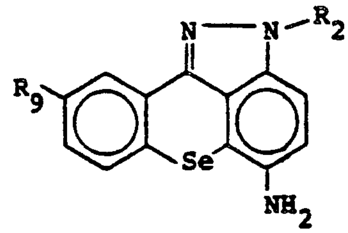

and the pharmaceutically acceptable salts thereof wherein:

$R_9$ is hydroxy or alkoxy of from 1 to 4 carbon atoms; and

$R_2$ is as defined in Claim 1.

7. A benzoselenino[4,3,2-*cd*]indazole compound according to Claim 1, having in free base form the following general formula:

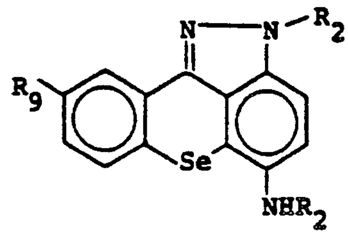

and the pharmaceutically acceptable salts thereof wherein:

$R_9$ is hydroxy or alkoxy of from 1 to 4 carbon atoms; and

$R_2$ is as defined in Claim 1.

8. A compound as claimed in Claim 1 and being chosen from:

*N,N*-diethyl-5-nitro-2*H*-[1]benzoselenino[4,3,2-*cd*]indazole-2-ethanamine;

5-amino-*N,N*-diethyl-2*H*-[1]benzoselenino[4,3,2-*cd*]indazole-2-ethanamine;

N-[2-[2-(diethylamino)ethyl]-2*H*-[1]benzoselenino[4,3,2-*cd*]indazol-5-yl]-1,2-ethanediamine;

N-[2-[2-(diethylamino)ethyl]-9-methoxy-2*H*-[1]benzoselenino[4,3,2-*cd*]indazol-5-yl]-1,2-ethanediamine;

2-[[2-[[2-[2-(diethylamino)ethyl]-2*H*-[1]-benzoselenino[4,3,2-*cd*]indazol-5-yl]-amino]ethyl]amino]-ethanol;

2-[[2-[5-[[2-[(2-hydroxyethyl)amino]ethyl]amino]-2*H*-[1]benzoselenino[4,3,2-*cd*]indazol-2-yl]-ethyl]-amino]ethanol;

*N,N*-diethyl-5-[[2-diethylamino)ethyl]amino]-2*H*-[1]-benzoselenino[4,3,2-*cd*]indazole-2-ethanamine;

*N*-[2-[2-(diethylamino)ethyl]-2*H*-[1]-benzoselenino[4,3,2-*cd*]indazol-5-yl]-1,3-propanediamine;

5-[(2-aminoethyl)amino]-2-[2-(1-pyrrolidinyl)ethyl]-2*H*-[1]benzoselenino[4,3,2-*cd*]indazole;

2-[2-(diethylamino)ethyl]-5-nitro-2*H*-[1]benzoselenino[4,3,2-*cd*]indazol-9-ol;

2-[2-(diethylamino)ethyl]-5-nitro-2*H*-[1]benzoselenino[4,3,2-*cd*]indazol-8-ol;

5-[(2-aminoethyl)amino]-2-[2-(diethylamino)ethyl]-2*H*-[1]benzoselenino[4,3,2-*cd*]indazol-9-ol;

5-[(2-aminoethyl)amino]-2-[2-(diethylamino)ethyl]-2*H*-[1]benzoselenino[4,3,2-*cd*]indazol-8-ol;

2-[2-(diethylamino)ethyl]-5-[[2-[(2-hydroxyethyl)amino]ethyl]amino]-2*H*-[1]benzoselenino[4,3,2-*cd*]-indazol-8-ol; or

2-[2-(diethylamino)ethyl]-5-[[2-[(2-hydroxyethyl)amino]ethyl]amino]-2*H*-[1]benzoselenino[4,3,2-*cd*]-indazol-9-ol.

9. A compound having the following general formula:

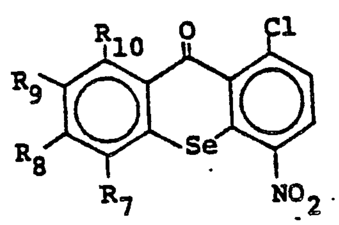

wherein $R_7$, $R_8$, $R_9$ and $R_{10}$ are independently hydrogen, hydroxy or alkoxy of from one to four carbon atoms.

10. A compound according to Claim 9, wherein $R_7$, $R_8$ and $R_9$ are hydrogen.

11. A compound according to Claim 9, wherein $R_7$, $R_9$ and $R_{10}$ are hydrogen.

12. A process for preparing a benzoselenino[4,3,2-*cd*]indazole compound having the following general formula:

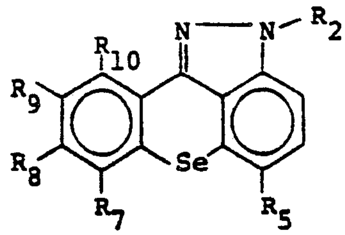

according to Claim 1, which process comprises:

reacting an appropriate 1-chloro-4-nitro-9*H*-selenoxanthen-9-one and an $R_2$-substituted hydrazine to form the compound as claimed in Claim 1 wherein $R_5$ is nitro;

if desired, converting said nitro compound, by reduction, to a compound as claimed in Claim 1, wherein $R_5$ is $NH_2$;

if further desired, converting the resulting compound wherein $R_5$ is $NH_2$ to a compound according to Claim 1 wherein $R_5$ is $NHR_2$ either (i) by alkylation with a haloalkylamine having the formula $XR_2$, optionally after covering sensitive groups with protecting groups, (ii) by reaction with an aldehyde or acetal, ketone or ketal and reducing the resulting Schiff base or (iii) by monoacylating with a reactive derivative of an acylating agent of formula R''''COOH and reducing the acylated product, optionally after covering sensitive groups with protecting groups; and

isolating the product in free base or acid addition salt form;

wherein X is chloro or bromo and $R_2$, R'''', $R_5$, $R_7$, $R_8$, $R_9$ and $R_{10}$ are as defined in Claim 1.

13. A process for preparing a benzoselenino[4,3,2-*cd*]indazole compound according to Claim 1, having in free base form the following structural formula:

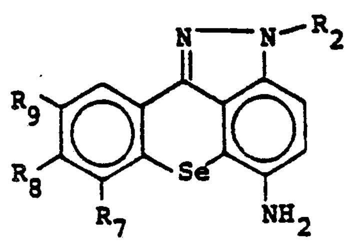

which process comprises reducing a compound having the structural formula:

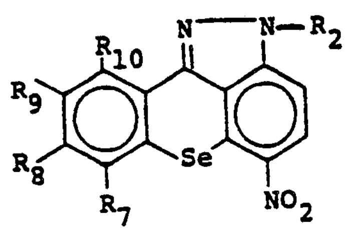

wherein $R_2$, $R_7$, $R_8$, $R_9$ and $R_{10}$ are as defined in Claim 1.

14. A pharmaceutical composition comprising a compound according to any one of Claims 1 to 8 in combination with a pharmaceutically acceptable carrier or diluent.

**Claims for the Contracting State: AT**

1. A process for preparing a benzoselenino[4,3,2-*cd*]indazole compound having in free base form the following general formula I:

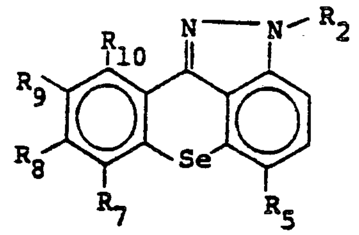

(I)

and the pharmaceutically acceptable salts thereof, wherein:

$R_7$, $R_8$, $R_9$ and $R_{10}$ independently represent hydrogen, hydroxy or alkoxy of from one to four carbon atoms;

$R_2$ is ANR'R'' in which A is a straight or branched alkylene chain of from two to five carbon atoms, optionally substituted with hydroxyl and in which R' and R'' are independently a hydrogen, a straight or branched alkyl of from one to four carbon atoms, optionally substituted with hydroxyl or R' and R'' when taken together, represent the bifunctional radical

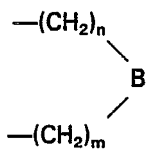

in which n and m are each, independently, the integer 2 or 3 and B is a direct bond or O, S, or NR''' wherein R''' is hydrogen or straight or branched alkyl of from one to four carbon atoms, optionally substituted with hydroxyl; and

$R_5$ is nitro, $NH_2$, $NHR_2$ or NHR'''' wherein R'''' is a one to four carbon atom acyl radical, a chloroacetyl radical,

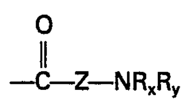

wherein Z is a straight or branched alkylene chain of from one to four carbon atoms and wherein $R_x$ and $R_y$ are each hydrogen, or a straight or branched alkyl of from one to four carbon atoms optionally substituted with hydroxy, or R'''' is the group

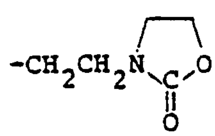

which process comprises:

reacting an appropriate 1-chloro-4-nitro-9*H*-selenoxanthen-9-one and an $R_2$-substituted hydrazine to form the compound of general formula I wherein $R_5$ is nitro;

if desired, converting said nitro compound, by reduction, to a compound of general formula I wherein $R_5$ is $NH_2$;

if further desired, converting the resulting compound wherein $R_5$ is $NH_2$ to a compound of general formula I wherein $R_5$ is $NHR_2$ either (i) by alkylation with a haloalkylamine having the formula $XR_2$, optionally after covering sensitive groups with protecting groups, (ii) by reaction with an aldehyde or acetal, ketone or ketal and reducing the resulting Schiff base or (iii) by monoacylating with a reactive derivative of an acylating agent of formula R''''COOH and reducing the acylated product, optionally after covering sensitive groups with protecting groups; and

isolating the product in free base or acid addition salt form;

wherein X is chloro or bromo and $R_2$, R'''', $R_5$, $R_7$, $R_8$, $R_9$ and $R_{10}$ are as defined above.

2. A process for preparing a benzoselenino[4,3,2-*cd*]indazole compound according to Claim 1, having in free base form the following structural formula:

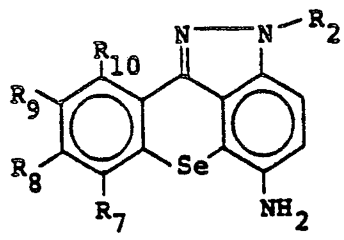

which process comprises reducing a compound having the structural formula:

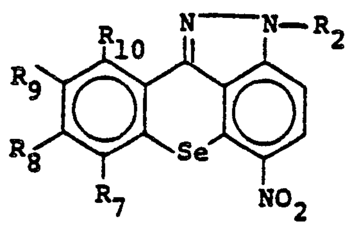

wherein $R_2$, $R_7$, $R_8$, $R_9$ and $R_{10}$ are as defined in Claim 1.

3. A process according to Claim 1, for preparing a compound having in free base form the following general formula:

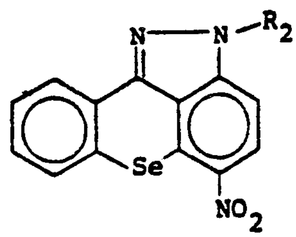

and the pharmaceutically acceptable salts thereof, wherein $R_2$ is as defined in Claim 1.

4. A process according to Claim 1, for preparing a compound having in free base form the following general formula:

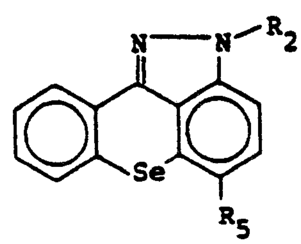

and the pharmaceutically acceptable salts thereof, wherein $R_2$ and $R_5$ are as defined in Claim 1.

5. A process according to Claim 4, wherein $R_2$ is diethylaminoethyl and $R_5$ is aminoethylamino.

6. A process according to Claim 1, for preparing a compound having in free base form the following general formula:

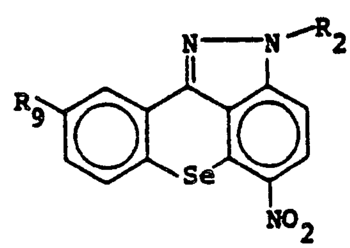

and the pharmaceutically acceptable salts thereof; wherein

$R_9$ is hydroxy or alkoxy of from one to four carbon atoms; and

$R_2$ is as defined in Claim 1.

7. A process according to Claim 1 or 2, for preparing a compound having in free base form the following general formula:

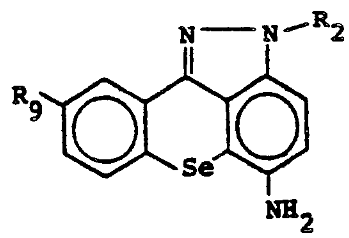

and the pharmaceutically acceptable salts thereof wherein:

$R_9$ is hydroxy or alkoxy of from 1 to 4 carbon atoms; and

$R_2$ is as defined in Claim 1.

8. A process according to Claim 1, for preparing a compound having in free base form the following general formula:

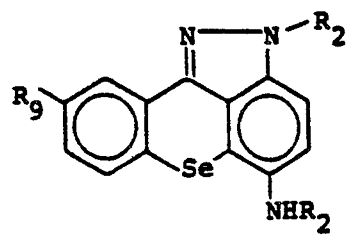

and the pharmaceutically acceptable salts thereof wherein:

$R_9$ is hydroxy or alkoxy of from 1 to 4 carbon atoms; and

$R_2$ is as defined in Claim 1.

9. A process according to Claim 1 for preparing one of the following compounds:

*N,N*-diethyl-5-nitro-2*H*-[1]benzoselenino[4,3,2-*cd*]indazole-2-ethanamine;

5-amino-*N,N*-diethyl-2*H*-[1]benzoselenino[4,3,2-*cd*]indazole-2-ethanamine;

N-[2-[2-(diethylamino)ethyl]-2*H*-[1]benzoselenino[4,3,2-*cd*]indazol-5-yl]-1,2-ethanediamine;

N-[2-[2-(diethylamino)ethyl]-9-methoxy-2*H*-[1]benzoselenino[4,3,2-*cd*]indazol-5-yl]-1,2-ethanediamine;

2-[[2-[[2-[2-(diethylamino)ethyl]-2*H*-[1]-benzoselenino[4,3,2-*cd*]indazol-5-yl]-amino]ethyl]amino]-ethanol;

2-[[2-[5-[[2-[(2-hydroxyethyl)amino]ethyl]amino]-2*H*-[1]-benzoselenino[4,3,2-*cd*]indazol-2-yl]-ethyl]-amino]ethanol;

*N,N*-diethyl-5-[[2-diethylamino)ethyl]amino]-2*H*-[1]-benzoselenino[4,3,2-*cd*]indazole-2-ethanamine;

*N*-[2-[2-(diethylamino)ethyl]-2*H*-[1]-benzoselenino[4,3,2-*cd*]indazol-5-yl]-1,3-propanediamine;

5-[(2-aminoethyl)amino]-2-[2-(1-pyrrolidinyl)ethyl]-2*H*-[1]benzoselenino[4,3,2-*cd*]indazole;

2-[2-(diethylamino)ethyl]-5-nitro-2*H*-[1]benzoselenino[4,3,2-*cd*]indazol-9-ol;

2-[2-(diethylamino)ethyl]-5-nitro-2*H*-[1]benzoselenino[4,3,2-*cd*]indazol-8-ol;

5-[(2-aminoethyl)amino]-2-[2-(diethylamino)ethyl]-2*H*-[1]benzoselenino[4,3,2-*cd*]indazol-9-ol;

5-[(2-aminoethyl)amino]-2-[2-(diethylamino)ethyl]-2*H*-[1]benzoselenino[4,3,2-*cd*]indazol-8-ol;

2-[2-(diethylamino)ethyl]-5-[[2-[(2-hydroxyethyl)amino]ethyl]amino]-2*H*-[1]benzoselenino[4,3,2-*cd*]-indazol-8-ol; or

2-[2-(diethylamino)ethyl]-5-[[2-[(2-hydroxyethyl)amino]ethyl]amino]-2*H*-[1]benzoselenino[4,3,2-*cd*]-indazol-9-ol.

10. A process for preparing a compound having the following general formula:

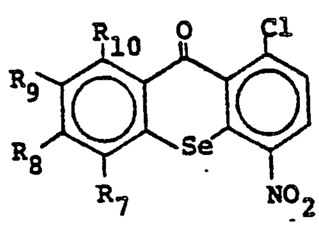

wherein $R_7$, $R_8$, $R_9$ and $R_{10}$ are independently hydrogen, hydroxy or alkoxy of from one to four carbon atoms;

which process comprises coupling an *o*-selenocyanobenzoic acid of formula

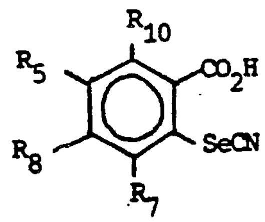

with 2,4-dichloronitrobenzene to give a compound of formula:

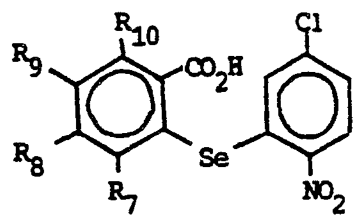

and non-oxidatively dehydrating the coupled compound.

11. A process according to Claim 10, wherein $R_7$, $R_8$ and $R_9$ are hydrogen.

12. A process according to Claim 10, wherein $R_7$, $R_9$ and $R_{10}$ are hydrogen.

13. A process for preparing a pharmaceutical composition, which process comprises combining a compound prepared in accordance with any one of Claims 1 to 9 together with a pharmaceutically acceptable carrier or diluent.

**Patentansprüche für die Vertragsstaaten: BE CH DE FR GB IT LI LU NL SE**

1. Benzoselenino[4,3,2-cd]indazolverbindung, welche in Form der freien Base die folgende allgemeine Formel I aufweist:

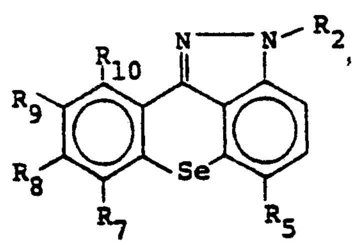

(I)

und die pharmazeutisch akzeptablen Salze davon, worin:

$R_7$, $R_8$, $R_9$ und $R_{10}$ unabhängig voneinander Wasserstoff, Hydroxy oder Alkoxy mit 1 bis 4 Kohlenstoffatomen repräsentieren;

$R_2$ ANR'R'' bedeutet, worin A eine gerade oder verzweigte Alkylenkette mit 2 bis 5 Kohlenstoffatomen, gegebenenfalls substituiert mit Hydroxyl, bedeutet, und worin R' und R'' unabhängig voneinander ein Wasserstoff, ein gerad- oder verzweigtkettiges Alkyl mit 1 bis 4 Kohlenstoffatomen, gegebenenfalls substituiert mit Hydroxyl, sind oder R' und R'', wenn sie zusammengenommen werden, das bifunktionelle Radikal

$$\begin{matrix} -(CH_2)_n & \\ & \diagdown \\ & & B \\ & \diagup \\ -(CH_2)_m & \end{matrix}$$

sind, worin jedes von n und m unabhängig voneinander eine ganze Zahl von 2 oder 3 ist, und B eine direkte Bindung oder O, S oder NR''', worin R''' Wasserstoff oder gerades oder verzweigtes Alkyl mit 1 bis 4 Kohlenstoffatomen, gegebenenfalls substituiert mit Hydroxyl, ist, darstellt; und

$R_5$ für Nitro, $NH_2$, $NHR_2$ oder NHR'''', worin R'''' ein Acylradikal mit 1 bis 4 Kohlenstoffatomen, ein Chloracetylradikal,

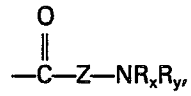

worin Z eine gerade oder verzweigte Alkylenkette mit 1 bis 4 Kohlenstoffatomen ist, und worin jedes von $R_x$ und $R_y$ Wasserstoff oder gerad- oder verzweigtkettiges Alkyl mit 1 bis 4 Kohlenstoffatomen, gegebenenfalls substituiert mit Hydroxy, ist, darstellt oder R'''' die Gruppe

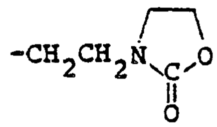

ist, steht.

2. Benzoselenino[4,3,2-*cd*]indazolverbindung nach Anspruch 1, welche in Form der freien Base die folgende allgemeine Formel aufweist:

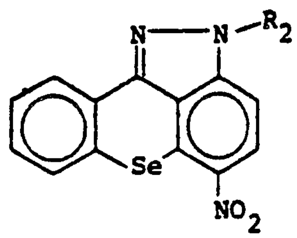

und die pharmazeutisch akzeptablen Salze davon, worin $R_2$ wie im Anspruch 1 definiert ist.

3. Benzoselenino[4,3,2-*cd*]indazolverbindung nach Anspruch 1, welche in Form der freien Base die folgende allgemeine Formel aufweist:

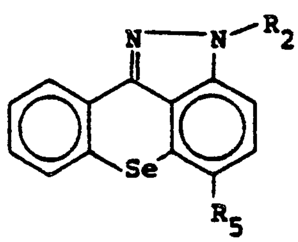

und die pharmazeutisch akzeptablen Salze davon, worin $R_2$ und $R_5$ wie im Anspruch 1 definiert sind.

4. Verbindung nach Anspruch 3, worin $R_2$ Diäthylaminoäthyl ist, und $R_5$ für Aminoäthylamino steht.

5. Benzoselenino[4,3,2-*cd*]indazolverbindung nach Anspruch 1, welche in Form der freien Base die folgende allgemeine Formel aufweist:

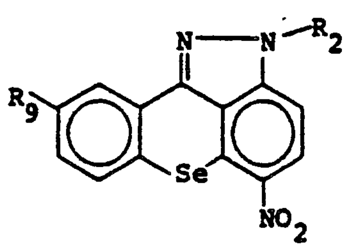

und die pharmazeutisch akzeptablen Salze davon, worin $R_9$ für Hydroxy oder Alkoxy mit 1 bis 4 Kohlenstoffatomen steht; und $R_2$ wie im Anspruch 1 definiert ist.

6. Benzoselenino[4,3,2-*cd*]indazol-Verbindung nach Anspruch 1, welche in Form der freien Base die folgende allgemeine Formel aufweist:

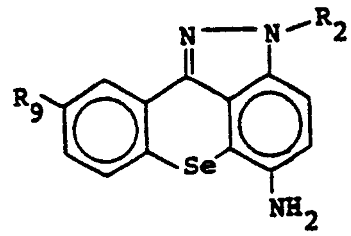

und die pharmazeutisch akzeptablen Salze davon, worin $R_9$ für Hydroxy oder Alkoxy mit 1 bis 4 Kohlenstoffatomen steht; und $R_2$ wie im Anspruch 1 definiert ist.

7. Benzoselenino[4,3,2-*cd*]indazol-Verbindung nach Anspruch 1, welche in Form der freien Base die folgende allgemeine Formel aufweist:

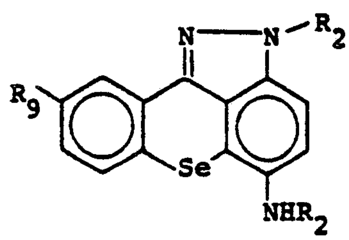

und die pharmazeutisch akzeptablen Salze davon, worin $R_9$ Hydroxy oder Alkoxy mit 1 bis 4 Kohlenstoffatomen ist; und $R_2$ wie im Anspruch 1 definiert ist.

8. Verbindung wie im Anspruch 1 beansprucht, welche ausgewählt ist aus:
*N,N*-Diäthyl-5-nitro-2*H*-[1]benzoselenino[4,3,2-*cd*]indazol-2-äthanamin;
5-amino-*N,N*-Diäthyl-2*H*-[1]benzoselenino[4,3,2-*cd*]indazol-2-äthanamin;
N-[2-[2-(Diäthylamino)-äthyl]-2*H*-[1]benzoselenino[4,3,2-*cd*]indazol-5-yl]-1,2-äthandiamin;
N-[2-[2-(Diäthylamino)-äthyl]-9-methoxy-2*H*-[1]benzoselenino[4,3,2-*cd*]indazol-5-yl]-1,2-äthandiamin;
2-[[2-[[2-[2-(Diäthylamino)-äthyl]-2*H*-[1]-benzoselenino[4,3,2-*cd*]indazol-5-yl]-amino]-äthyl]-amino]-äthanol;
2-[[2-[5-[[2-[(2-Hydroxyäthyl)-amino]-äthyl]-amino]-2*H*-[1]-benzoselenino[4,3,2-*cd*]indazol-2-yl]-äthyl]-amino]-äthanol;
*N,N*-Diäthyl-5-[[2-diäthylamino)-äthyl]-amino]-2*H*-[1]-benzoselenino[4,3,2-*cd*]indazol-2-äthanamin;
*N*-[2-[2-(Diäthylamino)-äthyl]-2*H*-[1]-benzoselenino[4,3,2-*cd*]indazol-5-yl]-1,3-propandiamin;
5-[(2-Aminoäthyl)-amino]-2-[2-(1-pyrrolidinyl)-äthyl]-2*H*-[1]-benzoselenino[4,3,2-*cd*]indazol;
2-[2-(Diäthylamino)-äthyl]-5-nitro-2*H*-[1]-benzoselenino[4,3,2-*cd*]indazol-9-ol;
2-[2-(Diäthylamino)-äthyl]-5-nitro-2*H*-[1]-benzoselenino[4,3,2-*cd*]indazol-8-ol;
5-[(2-Aminoäthyl)-amino]-2-[2-(diäthylamino)-äthyl]-2*H*-[1]-benzoselenino[4,3,2-*cd*]indazol-9-ol;
5-[(2-Aminoäthyl)-amino]-2-[2-(diäthylamino)-äthyl]-2*H*-[1]-benzoselenino[4,3,2-*cd*]indazol-8-ol;
2-[2-(Diäthylamino)-äthyl]-5-[[2-[(2-hydroxyäthyl)-amino]-äthyl]-amino]-2*H*-[1]-benzoselenino[4,3,2-*cd*]indazol-8-ol; oder
2-[2-(Diäthylamino)-äthyl]-5-[[2-[(2-hydroxyäthyl)-amino]-äthyl]-amino]-2*H*-[1]-benzoselenino[4,3,2-*cd*]indazol-9-ol.

9. Verbindung mit der folgenden allgemeinen Formel:

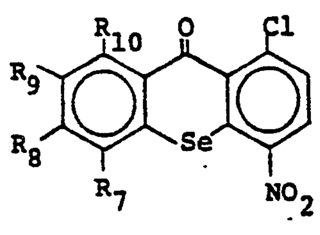

worin $R_7$, $R_8$, $R_9$ und $R_{10}$ unabhängig voneinander Wasserstoff, Hydroxy oder Alkoxy mit 1 bis 4 Kohlenstoffatomen sind.

10. Verbindung nach Anspruch 9, worin $R_7$, $R_8$ und $R_9$ Wasserstoff bedeuten.

11. Verbindung nach Anspruch 9, worin $R_7$, $R_9$ und $R_{10}$ Wasserstoff bedeuten.

12. Verfahren zur Herstellung einer Benzoselenino[4,3,2-*cd*]indazol-Verbindung mit der folgenden allgemeinen Formel:

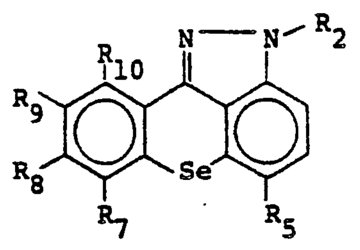

nach Anspruch 1, welches Verfahren umfaßt:

Umsetzen eines geeigneten 1-Chlor-4-nitro-9*H*-selenoxanthen-9-ons und eines $R_2$-substituierten Hydrazins zur Bildung der wie im Anspruch 1 beanspruchten Verbindung, worin $R_5$ für Nitro steht;

gewünschtenfalls Übeführen dieser Nitroverbindung mittels Reduktion in eine wie im Anspruch 1 beanspruchten Verbindung, worin $R_5$ für $NH_2$ steht;

wenn weiters gewünscht Überführen der resultierenden Verbindung, worin $R_5$ für $NH_2$ steht, in einer Verbindung nach Anspruch 1, worin $R_5$ für $NHR_2$ steht, entweder (i) durch Alkylieren mit einem Halogenalkylamin der Formel $XR_2$, gegebenenfalls nach dem Schützen von sensitiven Gruppen mit Schutzgruppen, (ii) durch Umsetzen mit einem Aldehyd oder Acetal, Keton oder Ketal und Reduktion der resultierenden Schiffschen Base oder (iii) durch Monoacylieren mit einem reaktiven Derivat eines Acylierungsmittels der Formel R''''COOH und Reduktion des acylierten Produkts, gegebenenfalls nach dem Schützen von sensitiven Gruppen mit Schutzgruppen; und

Isolieren des Produkts in Form der freien Base oder in Form des Säureadditionssalzes;

worin X für Chlor oder Brom steht, und $R_2$, R'''', $R_5$, $R_7$, $R_8$, $R_9$ und $R_{10}$ wie im Anspruch 1 definiert sind.

13. Verfahren zur Herstellung einer Benzoselenino[4,3,2-*cd*]indazol-Verbindung nach Anspruch 1, welche in Form der freien Base die folgende Strukturformel aufweist:

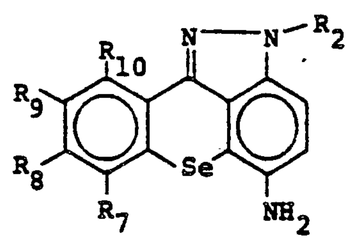

welches Verfahren die Reduktion einer Verbindung der Strukturformel

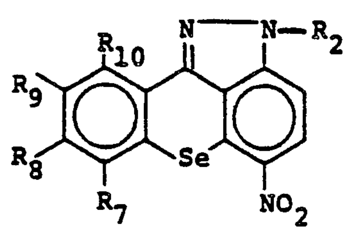

worin $R_2$, $R_7$, $R_8$, $R_9$ und $R_{10}$ wie im Anspruch 1 definiert sind, umfaßt.

14. Pharmazeutische Zusammensetzung, welche eine Verbindung nach irgendeinem der Ansprüche 1 bis 8 in Kombination mit einem pharmazeutisch akzeptablen Träger oder Verdünnungsmittel umfaßt.

**Patentansprüche für den Vertragsstaat: AT**

1. Verfahren zur Herstellung einer Benzoselenino[4,3,2-*cd*]indazolverbindung, welche in Form der freien Base die folgende allgemeine Formel I aufweist:

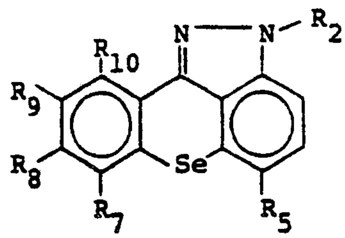

(I)

und der pharmazeutisch akzeptablen Salze davon, worin

$R_7$, $R_8$, $R_9$ und $R_{10}$ unabhängig voneinander Wasserstoff, Hydroxy oder Alkoxy mit 1 bis 4 Kohlenstoffatomen repräsentieren;

$R_2$ ANR'R'' bedeutet, worin A eine gerade oder verzweigte Alkylenkette mit 2 bis 5 Kohlenstoffatomen, gegebenenfalls substituiert mit Hydroxyl, bedeutet, und worin R' und R'' unabhängig voneinander ein Wasserstoff, ein gerad- oder verzweigtkettiges Alkyl mit 1 bis 4 Kohlenstoffatomen, gegebenenfalls substituiert mit Hydroxyl, sind, oder R' und R'', wenn sie zusammengenommen werden, das bifunktionelle Radikal

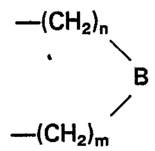

bedeuten, worin jedes von n und m unabhängig voneinander eine ganze Zahl von 2 oder 3 ist, und B eine direkte Bindung oder O, S oder NR''', worin R''' Wasserstoff oder gerades oder verzweigtes Alkyl mit 1 bis 4 Kohlenstoffatomen, gegebenenfalls substituiert mit Hydroxyl, ist, darstellt; und

$R_5$ für Nitro, $NH_2$, $NHR_2$ oder NHR'''', worin R'''' ein Acylradikal mit 1 bis 4 Kohlenstoffatomen, ein Chloracetylradikal,

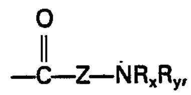

worin Z eine gerade oder verzweigte Alkylenkette mit 1 bis 4 Kohlenstoffatomen ist, und worin jedes von $R_x$ und $R_y$ Wasserstoff oder ein gerad- oder verzweigtkettiges Alkyl mit 1 bis 4 Kohlenstoffatomen, gegebenenfalls substituiert mit Hydroxy, ist, darstellt oder R'''' die Gruppe

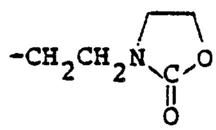

ist, steht;

welches Verfahren umfaßt:

Umsetzen eines geeigneten 1-Chlor-4-nitro-9*H*-selenoxanthen-9-ons und eines $R_2$-substituierten Hydrazins zur Bildung der Verbindung der allgemeinen Formel I, worin $R_5$ für Nitro ist;

gewünschtenfalls Überführen dieser Nitroverbindung durch Reduktion in eine Verbindung der allgemeinen Formel I, worin $R_5$ für $NH_2$ steht;

wenn weiters gewünscht, Überführen der resultierenden Verbindung, worin $R_5$ für $NH_2$ steht, in eine Verbindung der allgemeinen Formel I, worin $R_5$ für $NHR_2$ steht, entweder (i) durch Alkylieren mit einem Halogenalkylamin der Formel $XR_2$, gegebenenfalls nach dem Schützen von sensitiven Gruppen mit Schutzgruppen, (ii) durch Umsetzen mit einem Aldehyd oder Acetal, Keton oder Ketal und Reduktion der resultierenden Schiffschen Base oder (iii) durch Monoacylieren mit einem reaktiven Derivat eines Acylierungsmittels der Formel R''''COOH und Reduktion des acylierten Produkts, gegebenenfalls nach dem Schützen von sensitiven Gruppen mit Schutzgruppen; und

Isolieren des Produkts in Form der freien Base oder in Form von Säureadditionssalz;

worin X für Chlor oder Brom steht, und $R_2$, R'''', $R_5$, $R_7$, $R_8$, $R_9$ und $R_{10}$ wie oben definiert sind.

2. Verfahren zur Herstellung einer Benzoselenino[4,3,2-*cd*]indazolverbindung nach Anspruch 1, welche in Form der freien Base die folgende Strukturformel aufweist:

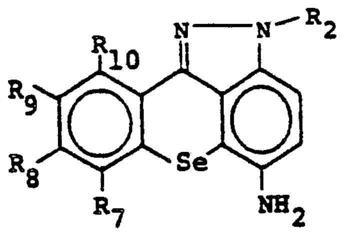

welches Verfahren die Reduktion einer Verbindung der Strukturformel:

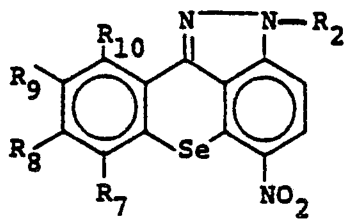

worin $R_2$, $R_7$, $R_8$, $R_9$ und $R_{10}$ wie im Anspruch 1 definiert sind, umfaßt.

3. Verfahren nach Anspruch 1 zur Herstellung einer Verbindung, welche in Form der freien Base die folgende allgemeine Formel aufweist:

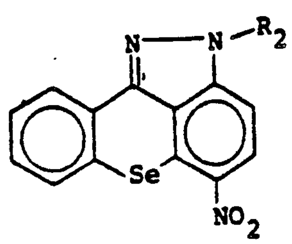

und der pharmazeutisch akzeptablen Salze davon, worin $R_2$ wie im Anspruch 1 definiert ist, umfaßt.

4. Verfahren nach Anspruch 1 zur Herstellung einer Verbindung, welche in Form der freien Base die folgende allgemeine Formel aufweist:

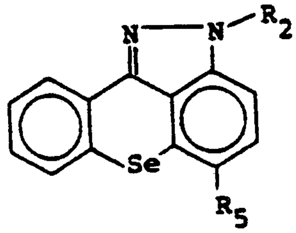

und der pharmazeutisch akzeptablen Salze davon, worin $R_2$ und $R_5$ wie im Anspruch 1 definiert sind.

5. Verfahren nach Anspruch 4, worin $R_2$ Diäthylaminoäthyl ist, und $R_5$ für Aminoäthylamino steht.

6. Verfahren nach Anspruch 1 zur Herstellung einer Verbindung, welche in Form der freien Base die folgende allgemeine Formel aufweist:

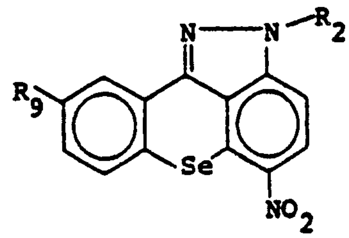

und der pharmazeutisch akzeptablen Salze davon; worin

$R_9$ für Hydroxy oder Alkoxy mit 1 bis 4 Kohlenstoffatomen steht; und

$R_2$ wie im Anspruch 1 definiert ist.

7. Verfahren nach Anspruch 1 oder 2 zur Herstellung einer Verbindung, welche in Form der freien Base die folgende allgemeine Formel aufweist:

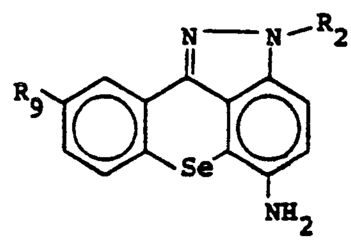

und der pharmazeutisch akzeptablen Salze davon; worin $R_9$ für Hydroxy oder Alkoxy mit 1 bis 4 Kohlenstoffatomen ist; und $R_2$ wie im Anspruch 1 definiert ist.

8. Verfahren nach Anspruch 1 zur Herstellung einer Verbindung, welche in Form der freien Base die folgende allgemeine Formel aufweist:

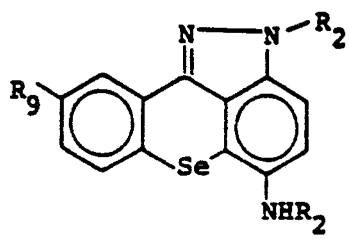

und die pharmazeutisch akzeptablen Salze davon; worin

$R_9$ Hydroxy oder Alkoxy mit 1 bis 4 Kohlenstoffatomen ist; und

$R_2$ wie im Anspruch 1 definiert ist.

9. Verfahren nach Anspruch 1 zur Herstellung einer der folgenden Verbindungen:

*N,N*-Diäthyl-5-nitro-2*H*-[1]benzoselenino[4,3,2-*cd*]indazol-2-äthanamin;

5-amino-*N,N*-Diäthyl-2*H*-[1]benzoselenino[4,3,2-*cd*]indazol-2-äthanamin;

N-[2-[2-(Diäthylamino)-äthyl]-2*H*-[1]benzoselenino[4,3,2-*cd*]indazol-5-yl]-1,2-äthandiamin;

N-[2-[2-(Diäthylamino)-äthyl]-9-methoxy-2*H*-[1]benzoselenino[4,3,2-*cd*]indazol-5-yl]-1,2-äthandiamin;

2-[[2-[[2-[2-(Diäthylamino)äthyl]-2*H*-[1]-benzoselenino[4,3,2-*cd*]indazol-5-yl]-amino]-äthyl]-amino]-äthanol;

2-[[2-[5-[[2-[(2-Hydroxyäthyl)-amino]-äthyl]-amino]-2*H*-[1]-benzoselenino[4,3,2-*cd*]indazol-2-yl]-äthyl]-amino]-äthanol;

*N,N*-Diäthyl-5-[[2-diäthylamino)-äthyl]-amino]-2*H*-[1]-benzoselenino[4,3,2-*cd*]indazol-2-äthanamin;

*N*-[2-[2-(Diäthylamino)-äthyl]-2*H*-[1]-benzoselenino[4,3,2-*cd*]indazol-5-yl]-1,3-propandiamin;

5-[(2-Aminoäthyl)-amino]-2-[2-(1-pyrrolidinyl)-äthyl]-2*H*-[1]-benzoselenino[4,3,2-*cd*]indazol;

2-[2-(Diäthylamino)-äthyl]-5-nitro-2*H*-[1]-benzoselenino[4,3,2-*cd*]indazol-9-ol;

2-[2-(Diäthylamino)-äthyl]-5-nitro-2*H*-[1]-benzoselenino[4,3,2-*cd*]indazol-8-ol;

5-[(2-Aminoäthyl)-amino]-2-[2-(diäthylamino)-äthyl]-2*H*-[1]-benzoselenino[4,3,2-*cd*]indazol-9-ol;

5-[(2-Aminoäthyl)-amino]-2-[2-(diäthylamino)-äthyl]-2*H*-[1]-benzoselenino[4,3,2-*cd*]indazol-8-ol;

2-[2-(Diäthylamino)-äthyl]-5-[[2-[(2-hydroxyäthyl)-amino]-äthyl]-amino]-2*H*-[1]-benzoselenino[4,3,2-*cd*]indazol-8-ol; oder

2-[2-(Diäthylamino)-äthyl]-5-[[2-[(2-hydroxyäthyl)-amino]-äthyl]-amino]-2*H*-[1]-benzoselenino[4,3,2-*cd*]indazol-9-ol.

10. Verfahren zur Herstellung einer Verbindung der folgenden allgemeinen Formel:

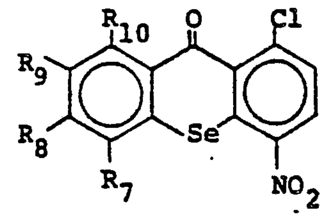

worin $R_7$, $R_8$, $R_9$ und $R_{10}$ unabhängig voneinander Wasserstoff, Hydroxy oder Alkoxy mit 1 bis 4 Kohlenstoffatomen sind; welches Verfahren das Koppen einer *o*-Selenocyano-benzoesäure der Formel

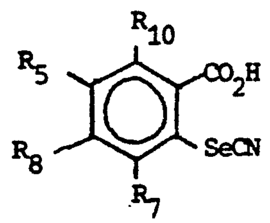

mit 2,4-Dichlornitrobenzol zum Erhalt einer Verbindung der Formel

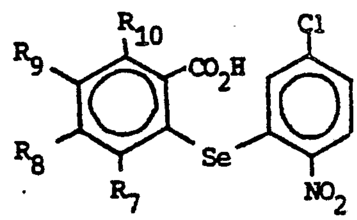

und die nicht-oxidierende Dehydratisierung der gekoppelten Verbindung umfaßt.

11. Verfahren nach Anspruch 10, worin $R_7$, $R_8$ und $R_9$ Wasserstoff sind.

12. Verfahren nach Anspruch 10, worin $R_7$, $R_9$ und $R_{10}$ Wasserstoff sind.

13. Verfahren zur Herstellung einer pharmazeutischen Zusammensetzung, welches Verfahren die Kombination einer nach irgendeinem der Ansprüche 1 bis 9 hergestellten Verbindung mit einem pharmazeutisch akzeptablen Träger oder Verdünnungsmittel umfaßt.

**Revendications pour les Etats contractants: BE CH DE FR GB IT LI LU NL**

1. Un composé benzosélénino[4,3,2-cd]indazole présentant sous forme de base libre, la structure représentée par la formule (I) suivante:

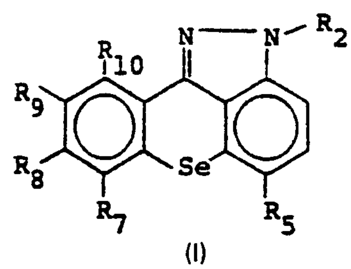

(I)

ainsi que leurs sels pharmaceutiquement acceptables, formule dans laquelle:

les substituants $R_7$, $R_8$, $R_9$ et $R_{10}$ représentent l'hydrogène, les groupes hydroxy ou alkoxy comportant 1 à 4 atomes de carbone,

$R_2$ et ANR'R'', où A est une chaîne alkylène linéaire ou ramifiée comportant 2 à 5 atomes de carbone, éventuellement substituée par un groupe hydroxyle;

R' et R'', sont, indépendamment l'un de l'autre, de l'hydrogène ou un radical alkyle à chaîne linéaire ou ramifiée comportant 1 à 4 atomes de carbone, éventuellement substitué par des groupes hydroxyles, R' et R'' pris simultanément pouvant être également sous la forme du radical bifonctionnel:

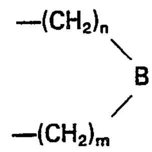

dans laquelle:

n et m sont des entiers égaux à 2 ou 3; et

B est une liaison directe ou O, S ou NR''', où R''' est de l'hydrogène ou un groupe alkyle linéaire ou ramifié comportant 1 à 4 atomes de carbone, éventuellement substitué par un groupe hydroxyle;

$R_5$ est un groupe nitro, $NH_2$, $NHR_2$, ou NHR'''' dans lequel R'''' est un radical alkyle comportant 1 à 4 atomes de carbone, chloroacétyle ou

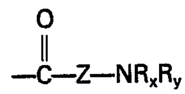

dans lequel:

$R_x$ and $R_y$ sont respectivement de l'hydrogène ou des radicaux alkyles linéaires ou ramifiés comportant 1 à 4 atomes de carbone, éventuellement substitués par un groupe hydroxyle; et

Z est une chaîne alkylène linéaire ou ramifiée comportant 1 à 4 atomes de carbone,

ou R'''' est le groupe:

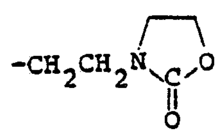

2. Un benzosélénino[4,3,2-cd]indazole selon la revendication 1, présentant, sous la forme de base libre, la formule générale suivante:

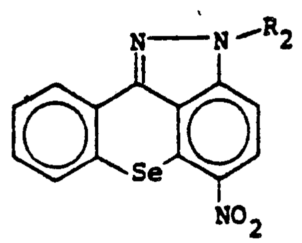

ainsi que ses sels pharmaceutiquement acceptables, formule dans laquelle $R_2$ est tel que défini dans la revendication 1.

3. Un benzosélénino[4,3,2-cd]indazole selon la revendication 1 présentant, sous la forme de base libre, la formule générale suivante:

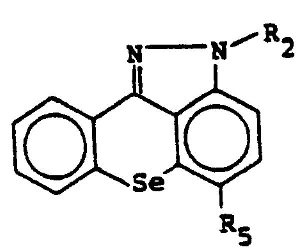

ainsi que ses sels pharmaceutiquement acceptables, formule dans laquelle $R_2$ et $R_5$ sont tels que définis dans la revendication 1.

4. Un composé selon la revendication 3, dans lequel:

$R_2$ est le radical diéthylaminoéthyl, et

$R_5$ est le radical aminoéthylamino.

5. Un benzoselenino[4,3,2-cd]indazole, selon la revendication 1, présentant sous sa forme de base libre, la formule générale suivante:

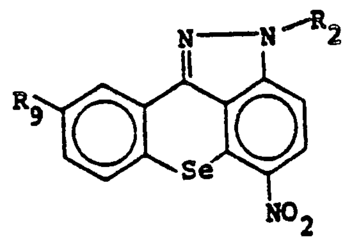

et ses sels pharmaceutiquement acceptables formule dans laquelle:

$R_9$ est un groupe hydroxy ou alkoxy comportant 1 à 4 atomes de carbone; et

$R_2$ est tel que défini dans la revendication 1.

6. Un benzosélénino[4,3,2-cd]indazole, selon la revendication 1, présentant sous sa forme de base libre, la formule générale suivante:

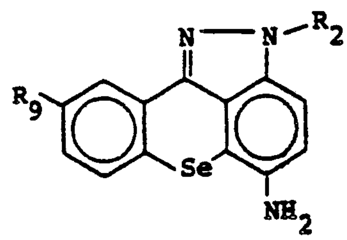

et ses sels acceptables du point de vue pharmaceutique, formule dans laquelle:

$R_9$ est groupe hydroxy ou alkoxy comportant 1 à 4 atomes de carbone; et

$R_2$ est tel que défini dans la revendication 1.

7. Un benzosélénino[4,3,2-cd]indazole, selon la revendication 1, présentant sous sa forme de base libre, la formule générale suivante:

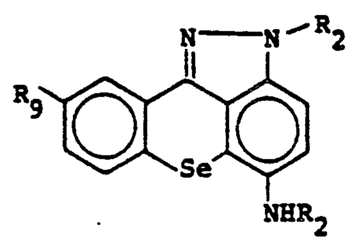

et ses sels acceptables du point de vue pharmaceutique, formule dans laquelle:

$R_9$ est groupe hydroxy ou alkoxy comportant 1 à 4 atomes de carbone; et

$R_2$ est tel que défini dans la revendication 1.

8. Un composé tel que défini dans la revendication 1 choisi dans le groupe comprenant:

N,N-diethyl-5-nitro-2H-[1]benzosélénino[4,3,2-cd]-indazol-2-éthanamine;

5-amino-N,N-diéthyl-2H-[1]benzosélénino[4,3,2-cd]-indazol-2-éthanamine;

N-[2-[2-(diéthylamino)éthyl]-2H-[1]-benzosélénino[4,3,2-cd]-indazol-5-yl]-1,2-éthanediamine;

N-[2-(diéthylamino)éthyl]-9-méthoxy-2H-[1]-benzosélénino[4,3,2-cd]-indazol-5-yl]-1,2-éthanediamine;

2-[[2-[[2-[2-(diéthylamino)éthyl]-2H-[1]-benzosélénino[4,3,2-cd]-indazol-5-yl]-amino]éthyl]amino]-éthanol;

2-[[2-[5-[[2-[(2-hydroxyéthyl)amino]éthyl]amino]-2H-[1]-benzosélénino[4,3,2-cd]-indazol-2-yl]-éthyl]-amino-éthanol;

N,N-diéthyl-5-[[2-diéthylamino)éthyl]amino]-2H-[1]-benzosélénino[4,3,2-cd]-indazol-2-éthanamine;

N-[2-[2-(diéthylamino)éthyl]-2H-[1]-benzosélénino[4,3,2-cd]-indazol-5-yl]-1,3-propanediamine;

5-[(2-aminoéthyl)amino]-2-[2-(1-pyrrolidinyl)éthyl]-2H-[1]-benzosélénino[4,3,2-cd]-indazole;

2-[2-(diéthylamino)éthyl]-5-nitro-2H-[1]-benzosélénino[4,3,2-cd]-indazol-9-ol;

2-[2-(diéthylamino)éthyl]-5-nitro-2H-[1]-benzosélénino[4,3,2-cd]-indazol-8-ol;

5-[(2-aminoéthyl)amino]-2-[2-(diéthylamino)éthyl]-2H-[1]-benzosélénino[4,3,2-cd]-indazol-9-ol;

5-[(2-aminoéthyl)amino]-2-[2-(diéthylamino)éthyl]-2H-[1]-benzosélénino[4,3,2-cd]-indazol-8-ol;

2-[2-(diéthylamino)éthyl]-5-[[2-[(2-hydroxyéthyl)-amino]éthyl]amino]-2H-[1]-benzosélénino[4,3,2-cd]-indazol-8-ol; ou

2-[2-(diéthylamino)éthyl]-5-[[2-[(2-hydroxyéthyl)-amino]éthyl]amino]-2H-[1]-benzosélénino[4,3,2-cd]-indazol-9-ol.

9. Un composé présentant la formule générale suivante:

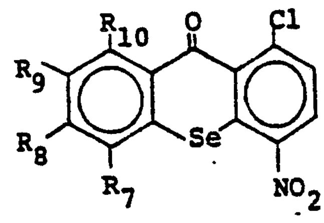

formule dans laquelle:

$R_7$, $R_8$, $R_9$ et $R_{10}$, sont, indépendamment l'un de l'autre, de l'hydrogène ou un groupe hydroxy ou alkoxy comportant 1 à 4 atomes de carbone.

10. Un composé selon la revendication 9, dans lequel $R_7$, $R_8$ et $R_9$ sont de l'hydrogène.

11. Un composé selon la revendication 9, dans lequel $R_7$, $R_9$ et $R_{10}$ sont de l'hydrogène.

12. Un procédé de préparation d'un benzosélénino[4,3,2-cd]-indazole, présentant la formule générale suivante:

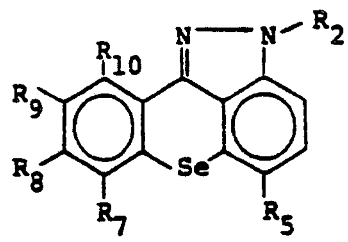

selon la revendication 1, lequel procédé comprend:

la réaction d'un 1-chloro-4-nitro-9H-sélénoxanthèn-9-one convenable et d'une hydrazine $R_2$-substituée, de façon à former le composé tel que revendiqué dans la revendication 1, $R_5$ étant un radical nitro;

si on le désire, la conversion du dérivé nitro par réduction en un composé tel que revendiqué dans la revendication 1, $R_5$ étant le radical $NH_2$;

si ultérieurement on le souhaite, la conversion du composé résultant dans lequel $R_5$ est $NH_2$ en un composé selon la revendication 5, $R_5$ étant $NHR_2$, soit par

(i) alkylation avec une haloalkylamine présentant la formule $XR_2$, éventuellement après protection des groupes sensibles avec des groupes protecteurs,

(ii) réaction d'un aldéhyde, d'un acétal, d'une cétone ou d'un cétal et réduction des produits résultants ou

(iii) monoacylation avec un dérivé réactif d'un agent d'acylation de formule R''''COOH et réduction du produit acylé résultant, éventuellement après protection des groupes sensibles par un groupe protecteur; et

isolation du composé résultant sous forme de base libre ou de sels d'addition d'acides;

formule dans laquelle:

X est chloro ou bromo; et

$R_2$ R'''', $R_5$, $R_7$, $R_8$, $R_9$ et $R_{10}$, sont tels que définis dans la revendication 1.

13. Un procédé de préparation de benzosélénino[4,3,2-cd]-indazole, selon la revendication 1, présentant sous sa forme de base libre la formule structurelle suivante:

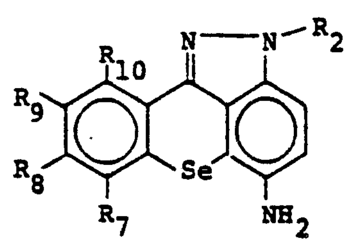

lequel procédé comprend la réduction d'un composé présentant la formule structurelle suivante:

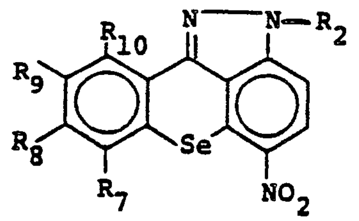

dans laquelle:

$R_2$ $R_7$, $R_8$, $R_9$ et $R_{10}$, sont tels que définis dans la revendication 1.

14. Une composition pharmaceutique comprenant un composé selon l'une des revendications 1 à 8 associé à un support, véhicule ou diluant acceptable du point de vue pharmaceutique.

**Revendications pour l'Etat contractant: AT**

1. Un procédé de préparation du benzosélénino[4,3,2-cd]-indazole ayant sous forme de base libre, la structure représentée par la formule (I) suivante:

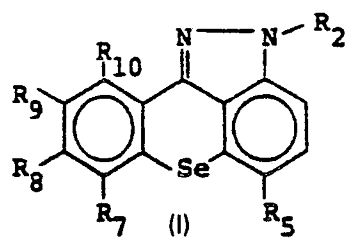

(I)

ainsi que leurs sels pharmaceutiquement acceptables, formule dans laquelle:

les substituants $R_7$, $R_8$, $R_9$ et $R_{10}$ représentent l'hydrogène, les groupes hydroxy ou alkoxy comportant 1 à 4 atomes de carbone,

$R_2$ et $ANR'R''$, où A est une chaîne alkylène linéaire ou ramifiée comportant 2 à 5 atomes de carbone, éventuellement substituée par un groupe hydroxyle;

R' et R'', sont, indépendamment l'un de l'autre, de l'hydrogène ou un radical alkyle à chaîne linéaire ou ramifiée comportant 1 à 4 atomes de carbone, éventuellement substitué par des groupes hydroxyles, R' et R'' pris simultanément pouvant être également sous la forme du radical bifonctionnel:

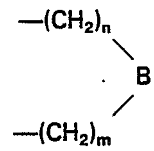

dans laquelle:

n et m sont des entiers égaux à 2 ou 3; et

B est une liaison directe ou O, S ou NR''', où R''' est de l'hydrogène ou un groupe alkyle linéaire ou ramifié comportant 1 à 4 atomes de carbone, éventuellement substitué par un groupe hydroxyle;

$R_5$ est un groupe nitro, $NH_2$, $NHR_2$, ou NHR'''' dans lequel R'''' est un radical alkyle comportant 1 à 4 atomes de carbone, chloroacétyle ou

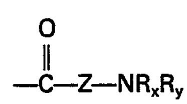

dans lequel:

$R_x$ and $R_y$ sont respectivement de l'hydrogène ou des radicaux alkyles linéaires ou ramifiés comportant 1 à 4 atomes de carbone, éventuellement substitués par un groupe hydroxyle; et

Z est une chaîne alkylène linéaire ou ramifiée comportant 1 à 4 atomes de carbone,

ou R'''' est le groupe:

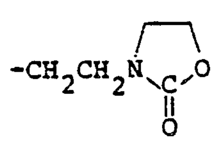

selon la revendication 1, lequel procédé comprend:

la réaction d'un 1-chloro-4-nitro-9H-sélénoxanthèn-9-one convenable et d'une hydrazine $R_2$-substituée, de façon à former le composé tel que revendiqué dans la revendication 1, $R_5$ étant un radical nitro;

si on le désire, la conversion du dérivé nitro par réduction en un composé tel que revendiqué à la revendication 1, $R_5$ étant le radical $NH_2$;

si ultérieurement on le souhaite, la conversion du composé résultant dans lequel $R_5$ est $NH_2$ en un composé selon la revendication 5, $R_5$ étant $NHR_2$, soit par

(i) alkylation avec une haloalkylamine présentant la formule $XR_2$, éventuellement après protection des groupes sensibles avec des groupes protecteurs,

(ii) réaction d'un aldéhyde, d'un acétal, d'une cétone ou d'un cétal et réduction des produits résultants ou

(iii) monoacylation avec un dérivé réactif d'un agent d'acylation de formule R''''COOH et réduction du produit acylé résultant, éventuellement après protection des groupes sensibles par un groupe protecteur; et

isolation du composé résultant sous forme de base libre ou de sels d'addition d'acides;

formule dans laquelle:

X est chloro ou bromo; et

$R_2$ R'''', $R_5$, $R_7$, $R_8$, $R_9$ et $R_{10}$, sont tels que définis dans la revendication 1.

2. Un procédé de préparation de benzosélénino[4,3,2-cd]-indazole selon la revendication 1, présentant, sous sa forme de base libre, la formule générale suivante:

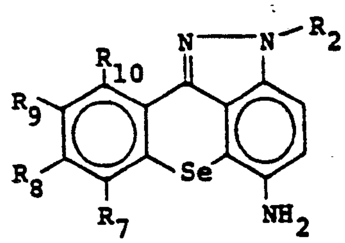

lequel procédé comprend la réduction du composé présentant la formule:

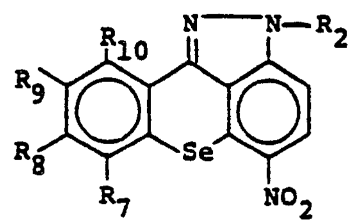

dans laquelle $R_2$, $R_7$, $R_9$ et $R_{10}$ sont tels que définis dans la revendication 1.

3. Un procédé, selon la revendication 1, de préparation d'un composé présentant, sous sa forme de base libre, la formule générale suivante:

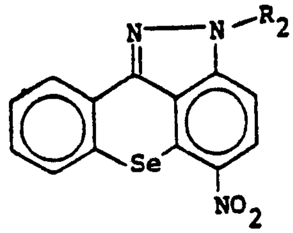

ainsi que ses sels pharmaceutiquement acceptables, formule dans laquelle $R_2$ est tel que défini dans la revendication 1.

4. Un procédé, selon la revendication 1, de préparation d'un composé présentant, sous sa forme de base libre, la formule générale suivante:

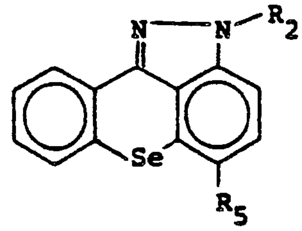

ainsi que ses sels pharmaceutiquement acceptables, formule dans laquelle $R_2$ et $R_5$ sont tels que définis dans la revendication 1.

5. Un procédé selon la revendication 4, dans laquelle $R_2$ est le radical diéthylaminoéthyle et $R_5$ est le radical aminoéthylamino.

6. Un procédé selon la revendication 1, de préparation d'un composé, présentant sous sa forme de base libre la formule générale suivante:

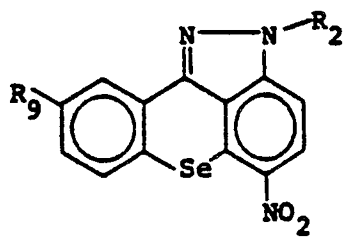

ainsi que ses sels pharmaceutiquement acceptables, formule dans laquelle $R_9$ est un groupe hydroxy ou alkoxy comportant 1 à 4 atomes de carbone et $R_2$ est tel que défini dans la revendication 1.

7. Un procédé selon les revendications 1 ou 2 de préparation d'un composé, présentant sous sa forme de base libre, la formule générale suivante:

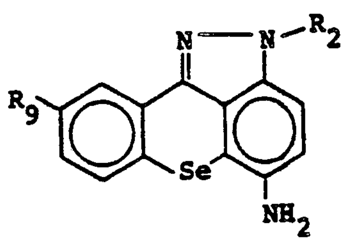

ainsi que ses sels pharmaceutiquement acceptables, formule dans laquelle $R_9$ est un groupe hydroxy ou alkoxy comportant 1 à 4 atomes de carbone et $R_2$ est tel que défini dans la revendication 1.

8. Un procédé selon la revendication 1 de préparation d'un composé présentant, sous sa forme de base libre, la formule générale suivante:

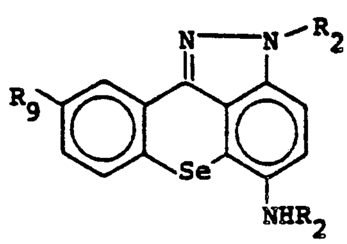

ainsi que ses sels pharmaceutiquement acceptables, formule dans laquelle $R_9$ est un groupe hydroxy ou alkoxy comportant 1 à 4 atomes de carbone et $R_2$ est tel que défini dans la revendication 1.

9. Un procédé selon la revendication 1 de préparation de l'un ou l'autre des composés suivants:

N,N-diethyl-5-nitro-2H-[1]benzosélénino[4,3,2-cd]-indazol-2-éthanamine;

5-amino-N,N-diéthyl-2H-[1]benzosélénino[4,3,2-cd]-indazol-2-éthanamine;

N-[2-[2-(diéthylamino)éthyl]-2H-[1]-benzosélénino[4,3,2-cd]-indazol-5-yl]-1,2-éthanediamine;

N-[2-[diéthylamino)éthyl]-9-méthoxy-2H-[1]-benzosélénino[4,3,2-cd]-indazol-5-yl]-1,2-éthanediamine;

2-[[2-[[2-[2-(diéthylamino)éthyl]-2H-[1]-benzosélénino[4,3,2-cd]-indazol-5-yl]-amino]éthyl]amino]-éthanol;
2-[[2-[5-[[2-[(2-hydroxyéthyl)amino]éthyl]amino]-2H-[1]-benzosélénino[4,3,2-cd]-indazol-2-yl]-éthyl]-amino]éthanol;
N,N-diéthyl-5-[[2-diéthylamino)éthyl]amino]-2H-[1]-benzosélénino[4,3,2-cd]-indazole-2-éthanamine;
N-[2-[2-(diéthylamino)éthyl]-2H-[1]-benzosélénino[4,3,2-cd]-indazol-5-yl]-1,3-propanediamine;
5-[(2-aminoéthyl)amino]-2-[2-(1-pyrrolidinyl)éthyl]-2H-[1]-benzosélénino[4,3,2-cd]-indazole;
2-[2-(diéthylamino)éthyl]-5-nitro-2H-[1]-benzosélénino[4,3,2-cd]-indazol-9-ol;
2-[2-(diéthylamino)éthyl]-5-nitro-2H-[1]-benzosélénino[4,3,2-cd]-indazol-8-ol;
5-[(2-aminoéthyl)amino]-2-[2-(diéthylamino)éthyl]-2H-[1]-benzosélénino[4,3,2-cd]-indazol-9-ol;
5-[(2-aminoéthyl)amino]-2-[2-(diéthylamino)éthyl]-2H-[1]-benzosélénino[4,3,2-cd]-indazol-8-ol;
2-[2-(diéthylamino)éthyl]-5-[[2-[(2-hydroxyéthyl)-amino]éthyl]amino]-2H-[1]-benzosélénino[4,3,2-cd]-indazol-8-ol; ou
2-[2-(diéthylamino)éthyl]-5-[[2-[(2-hydroxyéthyl)-amino]éthyl]amino]-2H-[1]-benzosélénino[4,3,2-cd]-indazol-8-ol.

10. Un procédé de préparation d'un composé présentant la formule générale suivante:

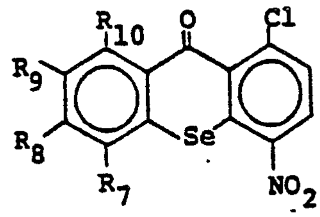

formule dans laquelle:
$R_7$, $R_8$, $R_9$ et $R_{10}$, sont, indépendamment l'un de l'autre, de l'hydrogène ou un groupe hydroxy ou alkoxy comportant 1 à 4 atomes de carbone;
lequel procédé consiste à coupler l'acide o-sélénocyanobenzoïque de formule:

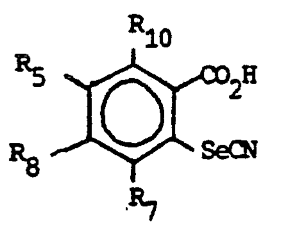

avec le 2,4-dichloronitrobenzène pour obtenir le composé présentant la formule:

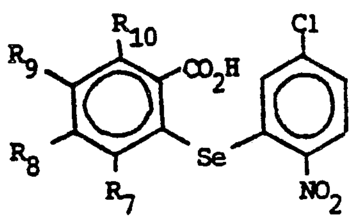

et à déshydrater de façon non oxydante le composé ainsi couplé.

11. Un procédé selon la revendication 10, dans lequel $R_7$, $R_8$ et $R_9$ sont de l'hydrogène.

12. Un procédé selon la revendication 10, dans lequel $R_7$, $R_9$ et $R_{10}$, sont de l'hydrogène.

13. Un procédé de préparation d'une composition pharmaceutique, lequel procédé comprend l'association du composé préparé selon une quelconque des revendications 1 à 9, avec un support, véhicule ou diluant acceptable du point de vue pharmaceutique.